# EUROPEAN PATENT APPLICATION

(11) **EP 4 806 984 A1**
(43) Date of publication of application: **16.09.2026**
(21) Application number: 25162741.0
(22) Date of filing: 10.03.2025
(51) Int. Cl.: C12M 3/00, C12M 1/12

(54) **CELL CULTURE CONSTRUCT FOR A CELL CULTURING SYSTEM**

(71) Applicant: Cellular Agriculture Ltd, Llanelli Carmarthenshire SA15 5TL (GB)
(72) Inventor: ALLAN, Scott James, Carnarthenshire, SA15 5TL (GB); ARGYLE, Iain, Carnarthenshire, SA15 5TL (GB); SHAUGHNESSY, Maxwell, Carmarthenshire, SA15 5TL (GB); ROBERTS, Nigel Somerville, Carrmarthenshire, SA15 5TL (GB); MIR FAKHAR, Moein, Carmarthenshire, SA15 5TL (GB); PARRY, Philippa, Carmarthenshire, SA15 5TL (GB); ELLIS, Marianne, Carnarthenshire, SA15 5TL (GB)
(74) Representative: HGF

(57) **Abstract**

The present disclosure provides a cell culture construct (102) for a cell culturing system (100). The cell culturing system (100) has a process vessel (104) with an interior volume and is adapted to receive the cell culture construct. The cell culture construct comprises a plurality of fibres (106) and an end support (108). First ends (110) of the plurality of fibres (106) are supported in the end support (108) such that the plurality of fibres extend from the end support and into the interior volume of the process vessel (104) when the cell culture construct is received in the process vessel. Second ends (112) of the plurality of fibres (106) are disposed within the interior volume of the process vessel (104).

## Description

The present invention relates to various aspects of culturing cells, for example for production of a food product or a biochemical product or a biopharmaceutical product.

### BACKGROUND

Cell culturing is a technique that is widely used in biological and medical research to understand cellular behaviour, investigate diseases, and develop treatments. Furthermore, cell culturing is employed in the production of vaccines, therapeutic proteins, and other biological products. Cell culturing is also used for the production of various biochemical products, for example ethanol.

Production of food by the in-vitro culturing of animal-derived cells, plant cells, fungal cells, bacterial cells, or algal cells is of increasing interest for food production. Lab-scale production of cultured food in its simplest form has been achieved, however scaling-up the process to make a viable economic product is still challenging.

It is known to use hollow fibre bioreactors for culturing cells by perfusion, where a cell culture medium is provided through the lumina of the hollow fibres to feed cells growing on external surfaces of the hollow fibres. Typically, such bioreactors are designed to maximise the number of fibres within the bioreactor by making the fibres as small as possible and by providing a high density of fibres within the bioreactor module. This increases the surface area for growing cells and for nutrient exchange, and so increases the production of cells. Accordingly, bioreactors are typically constructed with small diameter fibres packed at high density. It is also known to use solid fibres to provide support for cells adhered to the outer surfaces of the solid fibres.

Due to the large-scale requirements for commercial food production and increasingly for production of therapeutics, it is necessary to improve aspects of cell culturing, as well as the designs of any bioreactor systems used for such culturing.

### SUMMARY

According to an aspect of the present invention, there is provided a cell culture construct for a cell culturing system, the cell culturing system having a process vessel with an interior volume and being adapted to receive the cell culture construct, wherein the cell culture construct comprises a plurality of fibres and an end support, wherein first ends of the plurality of fibres are supported in the end support such that the plurality of fibres extend from the end support and into the interior volume of the process vessel when the cell culture construct is received in the process vessel, and wherein second ends of the plurality of fibres are disposed within the interior volume of the process vessel.

The second ends of the plurality of fibres are not connected to a further end support, and are therefore exposed to a fluid within the process vessel. In examples where the fibres are hollow, open-ended fibres, the lumina of the fibres are in fluid communication with the interior volume of the process vessel. In examples, the process vessel has a first vessel end and a second vessel end and the interior volume is defined between the first and second vessel ends.. The second ends of the fibres may be spaced from the vessel ends of the process vessel.

In examples, the second ends of the plurality of fibres are free to move within the process vessel. For example, the plurality of fibres may be unsupported within the process vessel, except at the first ends. In this way, the fibres can move within the interior volume of the process vessel, in particular in a fluid in the process vessel. In other examples, the fibres may be supported at a position along their length within the process vessel, for example by a tie or the like, to partially restrict their movement within the process vessel. In some examples, a tie may be provided between the first ends and the second ends, such that the second ends are free to move within the interior volume of the process vessel.

Advantageously, such a cell culture construct offers multiple advantages, including for construction and operation of the cell culture construct. For example, the cell culture construct provides for movement of the fibres within the process vessel during use, which beneficially improves cell seeding, culturing and harvesting. In particular, movement of the fibres within the process vessel improves fluid access and mixing amongst the fibres, which is beneficial during seeding, culturing and harvesting processes. Meanwhile, the fibres are joined at the end support, providing a unit that can be handled, moved, and processed with ease.

In some examples, the plurality of fibres may be hollow, open-ended fibres. Accordingly, during use a fluid flow may be generated through lumina of the fibres. In examples, cells may be cultured on internal or external surfaces of the hollow, open-ended fibres, or in an extracapillary volume within the process vessel. Use of hollow, open-ended fibres may beneficially permit a fluid flow to be generated within the process vessel with a low, controlled flow rate with little fluid shear acting on cells in the process vessel. The presence of the hollow, open-ended fibres in the process vessel may disturb fluid flow patterns and generate a more suffusive flow of fluid through the process vessel, reducing fluid shear acting on cells.

In other examples, the plurality of fibres may be solid fibres. In such examples, cells may be cultured on external surfaces of the solid fibres, or in an extracapillary volume within the process vessel. The presence of solid fibres within the process vessel may disturb fluid flow patterns and generate a more suffusive flow of fluid through the process vessel, reducing fluid shear acting on cells. The solid fibres also provide a large surface area for adherent cells.

In examples, the plurality of fibres may be inedible. As explained below, in some examples the cell culture construct is a food product cell culture construct used for culturing a food product. In such examples, the food product is separated from the fibres during harvesting.

### Fibres

In examples, the plurality of fibres each have an outer diameter of at least 0.3mm. In examples, the outer diameter of the fibres is between 0.3mm and 1 mm, for example between 0.4mm and 0.9mm, for example between 0.5mm and 0.8mm, for example one of 0.3mm, 0.4mm, 0.5mm, 0.6mm, 0.7mm, 0.8mm, 0.9mm or 1mm.

In examples, some of the fibres may have different outer diameters to others. Fibres with different outer diameters may be distributed evenly through the bundle of fibres in the end support, or concentrated in one or more locations within the end support, for example larger fibres may be concentrated towards a centre of the end support.

In examples, a fibre density of the plurality of fibres at the end support is between about 50 fibres/cm² and about 330 fibres/cm². Such a fibre density provides a balance between the generative capacity of the cell culture construct and the efficiency of seeding, culturing and harvesting cells.

In examples, the fibres are hollow, open-ended fibres and the fibre density of the fibres is between about 50 fibres/cm² and about 330 fibres/cm². In examples, the fibre density is between about 60 fibres/cm² and about 220 fibres/cm², for example between about 70 fibres/cm² and about 190 fibres/cm². In examples, the fibre density is greater than 120 fibres/cm², for example between 121 fibres/cm² and 330 fibres/cm², for example between 125 fibres/cm² and 220 fibres/cm², for example between 130 fibres/cm² and 190 fibres/cm².

In an example, the fibre diameter of the hollow, open-ended fibres is between 0.3mm and 1mm and the fibre density is preferably between about 50 fibres/cm² and about 330 fibres/cm², more preferably between about 60 fibres/cm² and about 220 fibres/cm², for example between about 70 fibres/cm² and about 190 fibres/cm². In examples, the fibre density may be greater than 120 fibres/cm², for example between 121 fibres/cm² and 330 fibres/cm², for example between 125 fibres/cm² and 220 fibres/cm², for example between 130 fibres/cm² and 190 fibres/cm².

In another example the fibre diameter of the hollow, open-ended fibres is between 0.5mm and 0.8mm and the fibre density is between about 50 fibres/cm² and about 220 fibres/cm², more preferably between about 60 fibres/cm² and about 170 fibres/cm², more preferably between about 70 fibres/cm² and about 140 fibres/cm².

In another example, the fibre diameter of the hollow, open-ended fibres is between 0.5mm and 0.7mm and the fibre density is between about 60 fibres/cm² and about 220 fibres/cm², more preferably between about 60 fibres/cm² and about 170 fibres/cm², more preferably between about 70 fibres/cm² and about 140 fibres/cm².

In examples, the fibre diameter of the hollow, open-ended fibres is 1mm and the fibre density is between about 50 fibres/cm² and about 70 fibres/cm².

In examples, the fibre diameter of the hollow, open-ended fibres is 0.3mm and the fibre density is between about 130 fibres/cm² and about 330 fibres/cm², more preferably between about 130 fibres/cm² and about 220 fibres/cm², more preferably between about 130 fibres/cm² and about 190 fibres/cm².

In examples, the fibre diameter of the hollow, open-ended fibres is 0.4mm and the fibre density is between about 110 fibres/cm² and about 300 fibres/cm², more preferably between about 110 fibres/cm² and about 180 fibres/cm², more preferably between about 110 fibres/cm² and about 150 fibres/cm².

In examples, the fibre diameter of the hollow, open-ended fibres is 0.5mm and the fibre density is between about 90 fibres/cm² and about 220 fibres/cm², more preferably between about 90 fibres/cm² and about 170 fibres/cm², more preferably between about 90 fibres/cm² and about 140 fibres/cm².

In examples, the fibre diameter of the hollow, open-ended fibres is 0.6mm and the fibre density is between about 70 fibres/cm² and about 170 fibres/cm², more preferably between about 80 fibres/cm² and about 140 fibres/cm².

In examples, the fibre diameter of the hollow, open-ended fibres is 0.7mm and the fibre density is between about 60 fibres/cm² and about 130 fibres/cm², more preferably between about 70 fibres/cm² and about 130 fibres/cm².

In examples, the fibre diameter of the hollow, open-ended fibres is 0.8mm and the fibre density is between about 50 fibres/cm² and about 110 fibres/cm², more preferably between about 60 fibres/cm² and about 110 fibres/cm², more preferably between about 70 fibres/cm² and about 110 fibres/cm².

In examples, the fibre diameter of the hollow, open-ended fibres is 0.9mm and the fibre density is between about 50 fibres/cm² and about 90 fibres/cm², more preferably between about 60 fibres/cm² and about 90 fibres/cm², more preferably about 80 fibres/cm².

In specific examples, the hollow, open-ended fibres may be arranged in a cross-sectional area of the end support of between about 7 cm² and about 180 cm². In such examples, the fibres may comprise between about 500 fibres and about 60,000 fibres.

In examples, the fibres are solid fibres and the fibre density is preferably between about 50 fibres/cm² and about 300 fibres/cm². In examples, the fibre density is between about 60 fibres/cm² and about 220 fibres/cm², for example between about 70 fibres/cm² and about 190 fibres/cm². In examples, the fibre density is greater than 120 fibres/cm², for example between 121 fibres/cm² and 300 fibres/cm², for example between 125 fibres/cm² and 220 fibres/cm², for example between 130 fibres/cm² and 190 fibres/cm².

In an example, the fibre diameter of the solid fibres is between 0.3mm and 1mm and the fibre density is preferably between about 50 fibres/cm² and about 300 fibres/cm². In examples, the fibre density of the solid fibres is between about 60 fibres/cm² and about 220 fibres/cm², for example between about 70 fibres/cm² and about 190 fibres/cm².

In another example the fibre diameter of the solid fibres is between 0.5mm and 0.8mm and the fibre density is between about 50 fibres/cm² and about 170 fibres/cm², more preferably between about 60 fibres/cm² and about 170 fibres/cm², more preferably between about 70 fibres/cm² and about 140 fibres/cm².

In another example, the fibre diameter of the solid fibres is between 0.5mm and 0.7mm and the fibre density is between about 60 fibres/cm² and about 170 fibres/cm², more preferably between about 60 fibres/cm² and about 180 fibres/cm², more preferably between about 70 fibres/cm² and about 140 fibres/cm².

In examples, the fibre diameter of the solid fibres is 0.3mm and the fibre density is between about 110 fibres/cm² and about 300 fibres/cm², more preferably greater than 120 fibres/cm², more preferably between about 120 fibres/cm² and about 220 fibres/cm², more preferably between about 121 fibres/cm² and about 220 fibres/cm², more preferably between about 130 fibres/cm² and about 190 fibres/cm².

In examples, the fibre diameter of the solid fibres is 0.4mm and the fibre density is between about 90 fibres/cm² and about 220 fibres/cm², more preferably between about 90 fibres/cm² and about 180 fibres/cm², more preferably between about 110 fibres/cm² and about 150 fibres/cm².

In examples, the fibre diameter of the solid fibres is 0.5mm and the fibre density is between about 70 fibres/cm² and about 170 fibres/cm², more preferably between about 80 fibres/cm² and about 170 fibres/cm², more preferably between about 90 fibres/cm² and about 140 fibres/cm².

In examples, the fibre diameter of the solid fibres is 0.6mm and the fibre density is between about 60 fibres/cm² and about 130 fibres/cm², more preferably between about 70 fibres/cm² and about 130 fibres/cm², more preferably between about 80 fibres/cm² and about 130 fibres/cm².

In examples, the fibre diameter of the solid fibres is 0.7mm and the fibre density is between about 60 fibres/cm² and about 110 fibres/cm², more preferably between about 70 fibres/cm² and about 130 fibres/cm².

In examples, the fibre diameter of the solid fibres is 0.8mm and the fibre density is between about 50 fibres/cm² and about 90 fibres/cm², more preferably between about 60 fibres/cm² and about 90 fibres/cm², more preferably between about 70 fibres/cm² and about 90 fibres/cm².

In examples, the fibre diameter of the solid fibres is 0.9mm and the fibre density is between about 50 fibres/cm² and about 70 fibres/cm², more preferably between about 60 fibres/cm² and about 70 fibres/cm².

In examples, the fibre diameter of the solid fibres is 1mm and the fibre density is between about 50 fibres/cm² and about 60 fibres/cm².

In specific examples, the fibres may be arranged in a cross-sectional area of the end support of between about 7 cm² and about 180 cm². In such examples, the plurality of fibres may comprise between about 350 fibres and about 54,000 fibres.

In examples, at a plane through the end support the fibres are arranged in a fibre distribution defined by an envelope. That is, the first ends of the fibres are arranged within the envelope at the end support. The envelope has a first dimension and a second dimension, and wherein a ratio of the first dimension to the second dimension is at least 2:1 in order to enhance fluid access to fibres within the fibre distribution.

Accordingly, within the cell culture construct the fibres are arranged in an envelope. The envelope may extend along the length of the cell culture construct (in a longitudinal direction perpendicular to the plane of the end support), and the fibres may be maintained within the envelope for the length of the cell culture construct.

In examples, the ratio of the first dimension to the second dimension is at least 2.5:1, for example at least 3:1, for example at least 3.5:1, for example at least 4:1, for example at least 5:1, for example at least 7:1, for example at least 8:1, for example at least 9:1, for example at least 10:1, for example at least 11:1, for example at least 12:1, for example at least 13:1, for example at least 14:1, for example at least 15:1, for example at least 16:1, for example at least 17:1, for example at least 18:1, for example at least 19:1, for example at least 20:1.

Such an aspect ratio between the first dimension and the second dimension creates an envelope of fibres that is relatively thin in one direction (second dimension), allowing greater access and penetration into the middle of the bundle of fibres, while also being relatively long in the other direction (first dimension) to provide scale. As explained further hereinafter, seeding, culturing and harvesting are each advantageously improved by the aspect ratio of the cell culture construct.

In examples, the ratio of the first dimension to the second dimension is at least 50:1, for example at least 100:1, for example at least 200:1, for example at least 300:1, for example at least 400:1, for example at least 500:1.

Advantageously, such ratios provide the advantages mentioned above and provide a larger scale cell culture construct for use in scaled-up manufacturing processes.

In examples, the ratio may be at most 500:1, for example at most 750:1, for example at most 1100:1. In preferred examples the ratio may be between 2:1 and 1000:1, for example between 2:1 and 500:1, for example between 2:1 and 100:1, for example between 2:1 and 50:1. In examples, the ratio may be between 3:1 and 500:1, for example between 3:1 and 100: 1, for example between 3:1 and 50:1. Advantageously, such ratios provide a balance between scale and practicality while still providing a ratio that provides one smaller dimension to improve access and penetration into the bundle of fibres.

In examples, the envelope is rectangular. The end support may also be rectangular. The fibres may be evenly or substantially evenly distributed through the envelope.

In examples, the envelope is an annulus formed between an outer boundary and an inner boundary, the second dimension being a distance between the inner boundary and the outer boundary, and wherein the first dimension is a length of the inner boundary or the outer boundary. If, during use, fluid flows from within the annulus to outside the annulus (through the envelope), then the first dimension is the length of the inner boundary. Alternatively if, during use, fluid flows from outside of the annulus to inside the annulus (through the envelope), then the first dimension is the length of the outer boundary. In some examples, as described hereinafter, fluid may flow in both directions during use (e.g., in an alternating direction), in which case both the inner boundary length and outer boundary length may be the first dimension. In examples, the lengths of the inner boundary and outer boundary both satisfy the aspect ratio(s) detailed above, ensuring fluid penetration into the fibres regardless of the direction of fluid flow relative to the annulus.

In examples, the inner boundary and/or the outer boundary is/are circular. The end support may also be circular. In examples, the inner boundary and/or the outer boundary is/are polygonal, for example triangular, square, rectangular, pentagonal, or hexagonal. The end support may have a polygonal shape corresponding to the outer boundary. The fibres may be evenly or substantially evenly distributed through the envelope.

In examples, the second dimension is less than 3cm, for example less than 2.5cm, for example less than 2cm, for example between 1cm and 3cm, for example between 1cm and 2.5cm, for example between 1cm and 2cm, for example between 1.1cm and 1.9cm, for example between 1.2cm and 1.8cm, for example between 1.3cm and 1.7cm, for example between 1.3cm and 1.5cm, for example about 1.4cm. Such a small second dimension ensures access and penetration into the bundle of fibres, for example by a fluid flow (for seeding, culturing and harvesting). Additionally, the small second dimension improves cell recovery during harvesting as cells can more easily escape the fibres after being detached, for collection.

In examples, the fibres are spaced from each other by at least 0.2mm, preferably between about 0.2mm and about 1mm, more preferably between about 0.3mm and about 0.6mm. Such spacings advantageously improve access and penetration into the fibre bundle, for example by a fluid flow or scraper, and also improve recovery of cells during harvesting as detached cells can more easily escape the fibres.

In other examples, the plurality of fibres may be edible. In such examples, when the cell culture construct is a food product cell culture construct, the fibres may form a part of the food product.

As described above, the fibres may be hollow or solid fibres. In examples, the hollow fibres may be porous. In examples, the hollow fibres may be non-porous.

In certain embodiments, the composition or compositions forming the one or more fibres comprises a polymer. In certain embodiments, the one or more fibres are biodegradable and/or edible.

Polymers that may be used to form the fibres may be any polymer suitable for culturing and/or maintenance of cells. The fibres may be formed from blends and mixtures of different polymers or may be predominantly formed from a single polymer type. Suitable polymers include biodegradable polymers. Alternatively, the polymers may be non-biodegradable or partially degradable. Biodegradable polymers are any polymers that may be broken down by biological systems, such as polymers that can be broken down into harmless products by the action of living organisms. The polymer may be a biocompatible polymer. Biocompatible polymers are, along with any metabolites or degradation products thereof, generally nontoxic to cells or to a recipient (such as a human or animal) and do not cause any significant adverse effects to cells or a recipient at concentrations resulting from the degradation of the polymers. Generally speaking, biocompatible polymers are polymers that do not elicit or result in negative effects on cell health or in a recipient. Edible materials are, by definition, biocompatible. As one use for the fibres described herein is the production of comestible products, biocompatible and/or biodegradable polymers may be advantageous if the fibres or part thereof is consumed (for example, ingested) by a person.

Biodegradable polymers include linear aliphatic polyesters such as polylactic acid, polyglycolic acid, polycaprolactone, polyhydroxybutyrate, polyhydroxyvalerate and their copolymers within the aliphatic polyester family such as poly(lactic-co-glycolic acid) and poly(glycolic acid-co-caprolactone); copolymers of linear aliphatic polyesters and other polymers such as poly(glycolic acid-co-trimethylene carbonate) copolymers, poly(lactic acid-co-lysine) copolymers, tyrosine-based polyarylates or polyiminocarbonates or polycarbonates, poly(lactide-urethane) and poly(ester-amide) polymers; polyanhydrides such as poly(sebacic anhydride); polyorthoesters such as 3,9-diethyidiene-2,4,8,10-tetraoxaspiro-5,5-undecane based polymers; poly(ester-ether) such as poly-p-dioxanone; polyamides, poly(amide-enamines) and poly(amido amine) dendrimers; and phosphorus-based polymers such as polyphosphazene and poly[bis(carboxy-lactophenoxy)] phosphazene.

The polymers may be edible polymers. Edible polymers refers to any polymer that is acceptable for use in an edible product or even that can be ingested without issue. Preferably, the edible polymers provide nutritional value.

Examples of edible polymers include polyvinyl alcohol, carboxyvinyl polymer, hydroxypropylmethylcellulose, hydroxyethylcellulose, methylcellulose, ethylcellulose, low-substituted hydroxypropyl cellulose, crystalline cellulose, carboxymethylcellulose sodium, a synthetic polymer compound such as carboxymethylcellulose calcium, carboxymethylcellulose and carboxymethylstarch sodium, sodium alginate, dextran, casein, pullulan, pectin, guar gum, xanthan gum, tragacanth gum, acacia gum, zein, gelatin, chitin and chitosan, silk, fibrin and polymer compounds obtained from natural products such as starch or soybean. Edible polymers may include polysaccharides, proteins from animal or plant sources. For example, such edible proteins may be derived from legumes such as chickpea, pea, soya and mung bean and may be formed into products such as Bean Curd Sheet. Edible polymers may also be derivable from waste food such as potatoes.

The use of an edible polymer may provide fibres and products, including the fibres which are edible. For example, a cell culture grown on the fibres may provide for an edible product that does not require the removal of the fibres before consumption and wherein the fibres provide nutritional value.

Suitable polymers further include synthetic polyamides, polycarbonates, polyisocyanurates (PIRs), polyurethanes, polyethers, polylactones, polylactams or glycols.

In some examples, the fibres include poly(lactic-co-glycolic acid) (PLGA). For example, the fibres may include 10% PLGA.

In some examples, the fibres include a reusable polymer. A reusable polymer refers to a polymer that does not degrade over time or due to use in culturing cells. Reusable polymers may provide a cell culture construct that can be washed and used multiple times. In some examples, the fibres are hydrophobic. In some examples, the fibres are washable. Washable refers to fibres that can be washed and/or sterilised without sustaining damage or loss of function. Fibres with such properties may reduce waste and costs.

**In** some examples, the polymer may comprise cellulose or cellulosic polymers or mixtures of both, polysulfone, polypropylene, acrylonitrile butadiene styrene (ABS), polycarbonate, polyethylene, or mixtures thereof.

**In** some examples, the polymer comprises polystyrene. The polymer can comprise polystyrene and a second polymer. The second polymer can be selected from the group consisting of, amongst others, polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The second polymer can comprise any suitable and compatible polymeric material. The composition can comprise polystyrene and a second polymer in a wt. % ratio of about 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 or 95:5.

**In** some examples, the polymer comprises polysulfone. The polymer can comprise polysulfone and a second polymer. The second polymer can be selected from the group consisting of polystyrene, polycarbonate, polypropylene, polyvinylidene fluoride, polycaprolactone, cellulose acetate, cellulose triacetate, polyethersulfone, polyphenylenesulfon, polymethyl methacrylate, acrylonitrile butadiene styrene or mixtures thereof. The second polymer can comprise any suitable and compatible polymeric material. The composition can comprise polysulfone and a second polymer in a wt. % ratio of about 5:95 to about 95:5 (e.g. about 10:90 to about 90:10), more preferably about 20:80 to about 80:20, and even more preferably about 25:75 to about 75:25. Polysulfone and the second polymer may be present in a wt. % ratio of 30:70, 35:65, 40:60, 45:55, 50:50, 55:45, 60:40, 65:35, 70:30.

Preferably, the polymer comprises polystyrene and/or polysulfone. Preferably, the polymer comprises polystyrene and polysulfone in a wt. % ratio of about 5:95 to about 95:5 (e.g. about 10:90 to about 90:10), more preferably about 20:80 to about 80:20, and even more preferably about 25:75 to about 75:25. Polystyrene and polysulfone may be present in a wt. % ratio of 30:70, 35:65, 40:60, 45:55, 50:50, 55:45, 60:40, 65:35, 70:30. Preferably, the composition comprises about 80 wt. % of polystyrene and about 20 wt. % of polysulfone, or about 60 wt. % of polystyrene and about 40 wt. %.

In some examples, the polymer comprises high impact polystyrene (HIPS). The polymer can comprise HIPS and a second polymer. The second polymer can be selected from the group consisting of polystyrene, polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The second polymer can comprise any suitable and compatible polymeric material. The composition can comprise HIPS and a second polymer in a wt. % ratio of about 5:95 to about 95:5 (e.g. about 10:90 to about 90:10), more preferably about 20:80 to about 80:20, and even more preferably about 25:75 to about 75:25. HIPS and the second polymer may be present in a wt. % ratio of 30:70, 35:65, 40:60, 45:55, 50:50, 55:45, 60:40, 65:35, 70:30.

Hollow fibres may be themselves be solid or semi-solid substrates having openings or apertures (pores), which allow components of a cell culture medium, such as metabolites, nutrients and gases (e.g. oxygen), to be delivered to cells attached to the outer surfaces of the hollow fibres.

Fibres described herein allow the growth of cells on the outer surface of the fibres. Thus the fibres described herein act as a 3-dimensional matrix that allows for the culture and maintenance of cells in a 3-dimensional architecture.

The fibres may have a Young's modulus of at least 1000 Pa. In some examples, the fibres have a Young's modulus from 1000 Pa to 1000000000 Pa. In some examples, the fibres have a Young's modulus between 8000 Pa to about 20000 Pa.

For example, the fibres may have a Young's modulus of around 115 MPa.

For hollow fibres, the lumina of each hollow fibre act as conduits to transport a cell culture medium to the cells located on the outer surface of the hollow fibres, as well as transporting waste products from the cells. The density of pores on each hollow fibre may be at least 1 pore/mm2. For example, at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300 pores/mm2.

In some examples, the pore density of the hollow fibres may be around 210 pores/mm².

Hollow fibres may be produced by any method, many of which are known in the field. For example, melting spinning, solution spinning, wet spinning, gel spinning, dry-wet spinning, liquid crystal spinning, dispersion spinning, reaction spinning and electrospinning.

**In** some examples, the hollow fibres may be fibres as described in Luetchford, Kim A., et al. "Next generation in vitro liver model design: Combining a permeable polystyrene membrane with a trans differentiated cell line." Journal of membrane science 565 (2018): 425-438, which is incorporated herein in its entirety.

### Cell Culturing System

According to a further aspect of the present invention, there is provided a cell culturing system comprising: a process vessel, and the cell culture construct described above, wherein the cell culture construct is received in the process vessel such that the plurality of fibres extend into the process vessel and the second ends of the plurality of fibres are free to move within the process vessel.

Advantageously, the cell culture construct provides for movement of the fibres within the process vessel during use, which beneficially improves cell seeding, culturing and harvesting. In particular, movement of the fibres within the process vessel improves fluid access and mixing amongst the fibres, which is beneficial during seeding, culturing and harvesting processes. Meanwhile, the fibres are joined at the end support, providing a unit that can be handled, moved, and processed with ease.

In examples, the process vessel comprises a first vessel end, a second vessel end, and a vessel side wall between the first vessel end and the second vessel end, and wherein the end support is disposed at the first vessel end. In examples, the end support may be attached or joined to the process vessel at the first vessel end. In some examples, the end support may seal an opening at the first vessel end, for example as a plug or cap closing an opening at the first vessel end.

In some examples, a single cell culture construct is received in the process vessel. In other examples, a plurality of cell culture constructs are received in the process vessel. The plurality of cell culture constructs may be arranged in a line or in an array. End supports of the plurality of cell culture constructs may abut each other or be spaced from each other. A support frame or the like may support the end supports at the first vessel end.

In examples, the process vessel has a vessel length between the first vessel end and the second vessel end. The plurality of fibres may have a length less than the vessel length. In this way, the second ends of the fibres are spaced from the second vessel end.

In examples, the cell culturing system comprises a fluid flow system operable to provide a fluid flow through the process vessel. The fluid flow system may include pipes, connectors, filters, pumps, reservoirs and the like for connecting to the cell culturing system and for generating the fluid flow. The fluid flow system may include one or more sensors for detecting one or more parameters of the fluid flow, within or externally of the process vessel, for example fluid temperature, flow rate, pH, dissolved oxygen, dissolved carbon dioxide, and the like. The cell culturing system may comprise a controller configured to control the fluid flow system and/or other components of the cell culturing system.

In examples, as mentioned above, the plurality of fibres comprise hollow, open-ended fibres. In such examples the fluid flow system may be adapted to generate a fluid flow through the plurality of fibres. The fluid flow may be from the first ends of the fibres to the second ends, or vice versa. The fluid flow direction in the fibres may be reversible, constant or variable, or intermittent.

In examples, the fluid flow system comprises a first port fluidly connected to lumina of the plurality of fibres to input fluid through the fibres. The first port may be connected to the end support, or at least partially formed by the end support.

In examples, the fluid flow system comprises a second port in communication with an internal volume of the process vessel. The second port may be disposed at or near the first vessel end, for example in the vessel side wall. In other examples, the second port is formed in the end support. For example, the second port may comprise a tube extending from the end support into the process vessel. The tube may extend from an external side of the end support to an internal side. In other examples, the second port may be disposed at or near the second vessel end. The fluid flow system may comprise a plurality of second ports.

In examples, the fluid flow system may be configured to generate a fluid flow in a direction between the first vessel end and the second vessel end, or vice versa. For example, the fluid flow system may input a fluid at the first port and extract a fluid at the second port, generating a fluid flow in a direction between the first vessel end and the second vessel end. The fluid flow may be generally parallel to the fibres. The fluid flow may be input into the process vessel through the fibres (hollow, open-ended fibres), and output from a second port in the first vessel end. In this example, the fluid flow is from the first ends of the fibres at the first vessel end, along the fibres to the second ends, then returning to the second port at the first vessel end. Such a fluid flow ensures that the circulated fluid reaches all parts within the process vessel. Such a flow pattern can be especially preferred as it can ensure a more uniform flow of nutrients to adherent cells on the external surfaces of the fibres during cell culturing. In other examples, the fluid flow is input at a second port at the second vessel end, and output at a port at the first vessel end. In this example the fluid flow may disturb, move, and/or deflect the fibres, creating mixing and separating the fibres, improving fluid flow into the fibre bundle.

In examples, the fluid flow system comprises side ports disposed in the vessel side wall. In such examples, the fluid flow system may be adapted to generate a fluid flow transverse to the fibres through the side ports. Such a transverse fluid flow may disturb, move, and/or deflect the fibres, creating mixing and separating the fibres, improving fluid flow into the fibre bundle.

In examples, the fluid flow system comprises one or more first ports at the first vessel end, and one or more second ports at the second vessel end, and the fluid flow system is configured to selectively generate a fluid flow through the ports, such that the direction of the fluid flow is changeable. The fluid flow system may additionally include side ports for generating further, different fluid flows.

In examples, the process vessel has a vessel length between the first vessel end and the second vessel end, and a vessel width in a transverse direction. The vessel width may increase and/or decrease between the first vessel end and the second vessel end. That is, the process vessel may get wider and/or narrower between the first vessel end and the second vessel end. Such a change in width of the process vessel will influence fluid flows and other interactions within the process vessel during use. For example, a region greater width may be provided to provide more space for movement of the fibres. In another example, the vessel width may narrow towards a port, which can generate an accelerated fluid flow as fluid is extracted at that port. Similarly, the vessel width may increase towards one end, which generate a decreasing fluid flow rate as fluid is extracted at a port in that end. In an example, the vessel width increases between the first vessel end and the second vessel end. The first vessel end may have a smaller vessel width, i.e., be narrower, than the second vessel end. Alternatively, the first vessel end may have a larger vessel width, i.e., be wider, than the second vessel end. In an example, the process vessel may comprise a greater vessel width at a position between the first vessel end and the second vessel end than either of the first vessel end or the second vessel end. That is, the vessel width may increase from the first vessel end to a position, and then narrow from the position to the second vessel end. In another example, the process vessel may comprise a smaller vessel width at a position between the first vessel end and the second vessel end than either of the first vessel end or the second vessel end. That is, the vessel width may decrease from the first vessel end to a position, and then widen from the position to the second vessel end, forming an hourglass shape. Different vessel width profiles may provide different flow characteristics in the process vessel, which may be beneficial for some fluid flow processes.

In examples, the vessel width is changeable. That is, the vessel width is changeable at at least one position along the process vessel. For example, the process vessel may be flexible and/or expandable. Moving the first vessel end towards or away from the second vessel end may flex the vessel side wall, causing the vessel width to change. In an example, the vessel side wall is flexible, for example a polymer bag, and can be positioned in a cavity mould that defines the form of the process vessel. The form of the process vessel can be changed by changing the cavity mould, for example by moving the process vessel to a different cavity mould.

In examples, the first vessel end may be moveable towards the second vessel end such that a distance between the first vessel end and the second vessel end is variable. For example, at least a part of the vessel side wall may be extendible (e.g., flexible, stretchable, or comprising folded portion (bellows)) allowing the first vessel end to be moved relative to the second vessel end.

In examples, the cell culturing system comprises a moving mechanism for moving the process vessel and/or the cell culture construct to generate movement of the plurality of fibres within the process vessel. The movement may be a vibrational, or reciprocal movement. Such movement may advantageously flex the fibres and mix the fluid and the fibres, causing movement of the fibres, mixing of the fluid, and penetration of the fluid into fibre bundle. The moving mechanism may be adapted to reciprocally move the process vessel and/or the cell culture construct. For example, the moving mechanism may move the first vessel end relative to the second vessel end (e.g., if the vessel side wall has an extendible portion). In other examples, the moving mechanism may be adapted to move the end support of the cell culture construct within the process vessel. For example, the end support may be slidable within the process vessel.

As described further below, the process vessel may be a culturing vessel and/or a harvesting vessel and/or a seeding vessel. That is, when the cell culture construct is received in the process vessel, any one or more of culturing, seeding and harvesting processes may be carried out.

In examples, a seeding method may be carried out in the process vessel to seed cells into the cell culturing system, in particular inside the process vessel and/or on the fibres. Similarly, a cell culturing method may be carried out in the process vessel, in which a cell culture medium is provided to culture the cells. The cell culturing method may be carried out in the same process vessel as the seeding method, or the cell culture construct may be moved from a seeding vessel to a culturing vessel. A harvesting method may be carried out during or after the cell culturing method to harvest cells and/or cell products. The harvesting method may be performed in the process vessel, which may be the same process vessel as the cell culturing method or a different harvesting vessel to which the cell culture construct is moved to for harvesting.

In some examples, the cell culturing system is a food product cell culturing system, and the cell culture construct is a food product cell culture construct. The cell culturing system may be for production of a cultured food product, for example a cultured meat product, a cultured plant product, a cultured fungal product, a cultured algal product, and/or a cultured bacterial product.

In other examples, the cell culturing system is a biochemical or biopharmaceutical product culturing system, and the cell culture construct is a biochemical or biopharmaceutical cell culture construct. The biochemical or biopharmaceutical product culturing system may be for production of a biochemical or biopharmaceutical product. The biochemical or biopharmaceutical product may be the cultured cells themselves, fragments or parts of the cultured cells, or a cell product expressed by the cultured cells. Such cell products may be proteins, enzymes, primary metabolites, and/or secondary metabolites.

In other examples, the cell culturing system may be for production of cosmetic, biomass, cell or fermentation products. Such products may be the cultured cells themselves, or proteins, enzymes, primary metabolites, and/or secondary metabolites.

The culturing of cells in the cell culture construct can be part of a fermentation process, such as a so-called "precision fermentation process". Such "precision fermentation" or "micro-fermentation" processes can be used to produce a wide range of materials by the culturing of modified organisms. In such processes, a feedstock is converted by the cultured cells, typically modified bacteria or yeasts, to produce a wide range of possible products or metabolites. Examples include the production of chemical feedstocks such as butanol and acetone by Clostridium Acetobutylicum and the production of proteins like casein and whey. In such processes, metabolites that are secreted into the surrounding media may be continuously extracted during recycling of media through the reactor or may be recovered once the culturing has completed. Yeasts, bacteria and other cell types used in the culturing methods may be modified, for example by genetic modification techniques.

The cell culturing system, in particular the fluid flow system, may comprise a pump system operable to circulate a fluid, for example a cell culture medium, through the process vessel.

The pump system may be any suitable pump. For example, the pump system may be a peristaltic pump system or vacuum pump system. The flow rate of cell culture medium that is flowed through the cell culturing system may be controlled by the pump system. The flow rate may be selected depending on the number of fibres being used, the cells being cultured and/or the number of cells seeded onto the outer surface of the fibres.

For example, cell culture medium may be pumped at a rate of at least 1 µL/hour/ fibre. That is to say, for each fibre used, the flow rate is 1 µL/hour. In some examples, the flow rate is at least 10 µL/hour/ fibre. For example, at least 10, 20, 30, 40, 50, 60, 70, 80, 90, 100, 200, 300, 400 µL/hour/ fibre. In some examples, the flow rate is from 100 to 300 µL/hour/ fibre. In some examples, the flow rate is between 240 to 280 µL/hour/ fibre. In some examples, the flow rate is about 267 µL/hour/ fibre.

The cell culturing system may further include a monitoring apparatus. Monitoring apparatus refers to any apparatus suitable for determining and/or monitoring changes in one or more properties of cells being cultured and/or one or more properties of the cell culture medium after having flowed through the cell culturing system (retentate). This can refer to media at different points within the body of the cell culturing vessel or even in the stream removing media from the cell culturing vessel.

For example, the monitoring apparatus may be a system for monitoring nutrient and/or metabolite content of the cell culture medium entering the cell culturing system (the permeate for examples with hollow fibres) and/or retentate. As used herein, in examples with hollow fibres "permeate" refers to cell culture medium that has passed through the pores of the hollow fibres (i.e. permeated through the hollow fibres). As used herein, retentate refers to cell culture medium that flows through the entire length of the lumen then the cell culturing system. Nutrients that may be monitored may be any nutrient and/or metabolite that is included in the cell culture medium used. For example, the nutrient and/or metabolite may be one or more of a saccharide (such as glucose), lactic acid (lactate), glutamine, glutamate, ammonia (ammonium ion), essential and non-essential amino acids, growth factors, albumin, attachment proteins, butanol, ethanol, acetone, acetic acid (acetate), vitamins, oxygen, and or carbon dioxide or the like.

The concentrations of nutrients and/or metabolites may be compared to the initial concentration of the corresponding nutrients and/or metabolites in the cell culture medium prior to culturing the cells or prior to being circulated through the cell culturing system. Changes in concentrations of nutrients and/or metabolites in the permeate and/or retentate in comparison to the initial cell culture medium may provide information on the rate of proliferation, cell number and/or condition (e.g. viability and/or health) of the cells being cultured.

In some examples, systems for monitoring concentrations of nutrients and/or metabolites may be separate from the cell culturing system. For example, the methods may further include collecting a portion of the permeate and/or retentate at predetermined time points and determining the concentrations of nutrients and/or metabolites. In some examples, the cell culturing system may include a system for monitoring nutrients and/or metabolites that is connected directly to the cell culturing system.

Suitable methods for determining concentrations of nutrients and/or metabolites in a cell culture medium are well known in the field. For example, kits such as those provided by Megazyme are available that include instructions therein. For example, for nutrients and/or metabolites such as saccharides (such as glucose), lactic acid (lactate), glutamine, glutamate, ammonia (ammonium ion). Alternatively or additionally HPLC and/or GC-MS may be used to determine concentrations of essential and non-essential amino acids, growth factors, albumin, attachment proteins. For example, monitors include those available from PreSens for oxygen and carbon dioxide.

In some examples, the monitoring apparatus may include a system for monitoring a concentration of dissolved oxygen in the permeate and/or retentate. The concentrations of dissolved oxygen may be compared to the initial concentration of dissolved oxygen in the cell culture medium prior to culturing the cells or prior to being circulated through the cell culturing system. Changes in concentrations of dissolved oxygen in the permeate and/or retentate in comparison to the initial cell culture medium may provide information on the rate of proliferation, cell number and/or condition (e.g. viability and/or health) of the cells being cultured.

Systems for determining the concentration of dissolved oxygen in cell culture medium are well known and include, for example, PreSens flow-through cell and PreSens Fibox 4 oxygen reader.

### Cell Culturing Method

According to a further aspect of the present invention, there is provided a cell culturing method for culturing cells in the cell culturing system described above, where the cell culturing system comprises a fluid flow system. The cell culturing method comprises: providing cells in the process vessel, and passing a cell culture medium through the process vessel to culture the cells.

The cell culturing method may comprise circulating fluid from the first ends of the plurality of fibres, to the second ends of the plurality of fibres, and extracting fluid from the process vessel.

In examples, the fluid flow through the process vessel may be input at the first port or the second port, and outlet at the first port or the second port. The first port and the second port may be in direct communication with an extracapillary space within the process vessel, in the space surrounding the fibres. In examples, the fibres may be hollow, open-ended fibres, and the fluid flow may pass through the fibres from the first ends to the second ends of the fibres, or vice versa. In one example, the fluid flow is input into the process vessel through the fibres, from the first ends to the second ends, and outlet from the extracapillary space of the process vessel at the first vessel end of the process vessel. In examples, the fluid flow may be input or output via side ports in the process vessel. The fluid flow may be in a direction parallel to the fibres and/or in a direction transverse to the fibres.

Advantageously, the width of the process vessel may vary between the first vessel end and the second vessel end, and/or be changeable, in order to vary and/or control the flow pattern of the cell culture medium during the cell culturing method.

In certain embodiments, the cell culturing method also includes maintaining the process vessel (and cells) at a temperature of at least 15°C. In embodiments, the method includes maintaining a CO₂ content of about 5% CO₂.

In certain embodiments, the cell culture medium is circulated through the cell culturing system for at least 3 hours. In certain embodiments, a first cell culture medium is first circulated through the cell culturing system, optionally wherein the first cell culture medium is a proliferation medium. In certain embodiments, the cell culturing method further comprises circulating a second cell culture medium through the cell culturing system, optionally wherein the second cell culture medium is a differentiation medium; further optionally wherein the differentiation media is circulated through the cell culturing system for at least 3 hours.

In certain embodiments, the cell culturing system comprises apparatus for monitoring metabolite concentration and/or oxygen concentration of the culture media.

The cell culture medium that may be used in the methods described herein may be any suitable cell culture medium. The cell culture medium may be selected depending on the type of cell cells being cultured. Examples of cell culture media that may be used include minimal essential medium (MEM, Sigma, St. Louis, Mo); Dulbecco's modified Eagle medium (DMEM, Sigma); Ham F10 medium (Sigma); Cell culture medium (HyClone, Logan, Utah); RPMI-1640 culture media (Sigma); and chemical-defined (CD) culture media (which are formulated for individual cell types), such as CD-CHO culture media (Invitrogen, Carlsbad, Calif). Such media are typically suitable for culturing animal cells. Media such as McCowns Woody Plant Medium, Murashige & Skoog media and Linsmaier & Skoog media are used for plant cell culturing. In examples, the cell culture medium may be pharmaceutical-grade, or food-grade media. The culture solution described above can be supplemented with auxiliary components or contents as needed, for example to change the properties or behaviours of the cells. This includes any component of the appropriate concentration or amount required or desired.

In some examples, the cell culture medium may be configured to control, for example to facilitate, proliferation, differentiation, and/or maturation. Different media and/or physical stimuli may be used at different stages of the cell culturing process.

The cell culture medium described above can be supplemented with auxiliary components or contents as needed. The cell culture medium may include one or more additives such as antibiotics, proteins, amino acids and/or sugars.

"Medium" and "cell culture medium" refer to a nutrient source used for growing or maintaining cells. As is understood by a person of skill in the art, the nutrient source may contain components required by the cell for growth and/or survival or may contain components that aid in cell growth and/or survival. Vitamins, essential or non-essential amino acids, trace elements, and surfactants (e.g., poloxamers) are examples of medium components. Any media provided herein may also be supplemented with any one or more of insulin, plant hydrolysates and animal hydrolysates.

"Culturing" a cell refers to contacting a cell with a cell culture medium under conditions suitable for the viability and/or growth and/or proliferation, differentiation and maturation of the cell.

Perfusing the cell culture medium may include perfusing a first cell culture medium and then subsequently perfusing one or more second cell culture media. The first cell culture medium may be a cell culture medium that is for proliferating cells and may be referred to as proliferation medium.

Proliferation medium may be a medium comprising a source of nutrients, such as vitamins, minerals, carbon and energy sources, and other beneficial compounds that facilitate the biochemical and physiological processes occurring during expansion or proliferation of cells. The proliferation medium may comprise one or more carbon sources, vitamins, amino acids, and inorganic nutrients. Representative carbon sources include monosaccharides, disaccharides, and/or starches. For example, the proliferation medium may contain one or more carbohydrates such as sucrose, fructose, maltose, galactose, mannose, and lactose. The proliferation medium may also comprise amino acids. Suitable amino acids may include amino acids commonly found incorporated into proteins as well as amino acids not commonly found incorporated into proteins, such as arginosuccinate, citrulline, canavanine, ornithine, and D-stereoisomers. The proliferation medium may also comprise growth promotion agents, such as serum, for example, adult bovine serum (ABS). Examples of other growth promotion agents include growth factors (e.g. recombinant growth factors), bovine ocular fluid, sericin protein, earthworm heat inactivated coelomic fluid. In some examples, the proliferation media may be a serum free culture media and may optionally include additional components depending on the cells being cultured. For example, serum free culture medias include those commercially available from ThermoFisher, Lonza Bioscience and Merck. The proliferation medium may also comprise antibiotics.

For example, the proliferation medium may be Dulbecco's Modified Eagle's Medium (DMEM), which may include 10% (V/V) filter sterilised adult bovine serum and 1% (V/V) penicillin/streptomycin solution.

In some examples, such as when the cells are derived from an insect, the proliferation media may be a media such as Gibco insect media available from ThermoFisher.

The cells may be maintained and cultured in proliferation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours or even longer, such as 200 hours or 300 hours or 500 hours. In some examples, the cells may be maintained in proliferation media continuously.

The cell culture medium may be changed to a second cell culture medium. The second cell culture medium may be a differentiation medium. Differentiation medium refers to a medium designed to support the differentiation of cells, that is, supporting the process of a cell changing from one cell type to another. The differentiation medium may include one or more amino acids, antibiotics, vitamins, salts, minerals, or lipids. The differentiation medium may include at least one carbon source, such as a sugar. For example, glucose. The differentiation medium may include one or more proteins, amino acids or other additional acids. In some examples, the differentiation media includes one or more growth promotion agents, such as serum, for example, adult bovine serum or horse serum. Examples of other growth promotion agents include growth factors (e.g. recombinant growth factors), bovine ocular fluid, sericin protein, earthworm heat inactivated coelomic fluid. In some examples, the differentiation media may be a serum free culture media and may optionally include additional components depending on the cells being cultured. For example, serum free culture medias include those commercially available from ThermoFisher, Lonza Bioscience and Merck. In some examples, the differentiation medium may be high-glucose DMEM (97%) supplemented with 2% horse serum and 1% penicillin/streptomycin solution.

The cells may be maintained and cultured in differentiation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours or even longer, such as 200 hours or 300 hours or 500 hours.

In some examples, the cell culture medium may be changed a number of times. For example, the cells may first be cultured in proliferation media; then, the cell culture medium is switched to differentiation media. The differentiation media may then be switched to proliferation media. The proliferation media may be the same as before or may contain different components and/or concentrations of components to the initial proliferation media.

In some examples, the cells may be continuously cultured. That is to say that the cells are maintained in one or more cell culture media as described herein. The cells may be cultured for a first time period (e.g. at least 3 hours or more) in a first media, then for a second time period (e.g. at least 3 hours or more) in a second media and then cultured in a further media and continuously cultured with any number of changes of the culture media so that cells can be constantly proliferated and/or differentiated. In such examples, the cells may be harvested at predetermined cell densities or time points in order to avoid overcrowding, reduced viability and/or cell death. In other such examples, a cell product may be harvested, leaving the cells themselves in situ.

In examples, the cell culturing method may be manual, automated, or semi-automated. In particular, the addition and changing of cell culture medium may be carried out manually, or in an automated or semi-automated manner.

The cell culturing conditions (e.g. for either or both proliferation or differentiation) may be selected depending on the cell type and/or cell source. In some examples, cells may be cultured (e.g. proliferated and/or differentiated) at a temperature of at least 5°C. In some examples, the cells may be cultured at a temperature of at least 5°C, 10°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, or 37°C.

For example, for culturing of insect cells, the cells may be cultured at a temperature of from 15°C to 32 °C.

For example, for culturing mammal cells, the cells may be cultured at a temperature of 37°C.

For example, for marine animal cells (such as crustaceans, fish or mollusc cells), cells may be cultured at a temperature of from 5°C to 35°C, or from 15°C to 32 °C. In some examples, marine animal cells may be cultured at a temperature between 15°C to 30 °C.

The cells may be cultured in defined atmospheric conditions. For example, the cells may be cultured in an atmosphere that has predetermined humidity and/or gas concentration. For example, cells may be cultured in an atmosphere including at least 5% CO2.

In examples, the cell culturing method may comprise a wash and/or sterilisation step preceding the seeding of cells to the cell culturing system.

The perfusion culturing method described above may be particularly suited to culturing muscle cells to form a cultured meat product. However, the perfusion culturing method described above may also be used to culture other kinds of cells for production of cells or cell products. This includes the culturing of plant cells, fungal cells, bacterial cells, and/or algal cells, for the production of food. It may also include the culturing of cells for biochemical products, biopharmaceutical products, cosmetic products, biomass products, or fermentation products.

In various examples, any suitable culturing method can be used. Examples thereof for plants include dedifferentiation (callusing) and redifferentiation of plant tissue, anther culture, shoot apex culture, protoplast culture, batch culture, co-culture, clonal cell culture, seed culture, an ovary culture method, an ovule culture method, an embryo culture method and a pollen culture method.

### Harvesting System and Harvesting Method

According to a further aspect of the present invention, there is also provided a harvesting method for harvesting cells and/or cell products from the cell culturing system described above. The harvesting method comprises generating a fluid flow through the process vessel and collecting cells and/or cell products.

In examples, the fluid flow is in a direction transverse to the plurality of fibres. For example, the fluid flow may be generated at one or more side ports in the process vessel, which direct the fluid flow in a direction transverse to the fibres. Such a fluid flow will apply fluid shear to cells on the fibres and within the bundle of fibres, which may cause cell detachment and/or entrainment for collecting the cells. The transverse fluid flow will also move and deform the fibres, further improving detachment and/or entrainment of cells.

In other examples, the fluid flow is in a direction parallel to the plurality of fibres. For example, the fluid flow may be from the second ends towards the first ends of the plurality of fibres. In other examples, the fluid flow may be from the first ends towards the second ends of the plurality of fibres. Such a flow along the external surfaces of the fibres may apply fluid shear to cells adhered to the fibres that may increase cell detachment. The fluid flow will also move and deform the fibres, further improving detachment. The fluid flow will also entrain detached (or not attached) cells, allowing them to be collected.

In other examples, the plurality of fibres comprise hollow, open-ended fibres and the fluid flow is provided through the plurality of fibres. In some examples the cells are cultured within lumina of the fibres, in which case the fluid flow through the fibres will dislodge and entrain the cells for collection. In other examples, the cells are cultured on the external surfaces of the fibres, in which case the fluid flow through the fibres may cause cell detachment by applying pressure through the walls of the fibres (if the fibres are porous), and/or by deforming or moving the fibres due to the increased pressure in the lumina of the fibres. In other examples, providing the fluid flow through the fibres may create a controlled, low shear fluid flow for detaching and/or entraining cells or cell products in the process vessel, allowing them to be removed from the process vessel, for example at an outlet port.

In examples, the fluid flow may comprise a cell detachment agent. The cell detachment agent may be chemical or biological and configured to reduce cell adhesion to the fibres or other structure to which the cells are adhered.

In examples, the harvesting method may include moving the cell culture construct relative to the process vessel. In particular, the harvesting method may comprise moving the fibres relative to a fluid in the process vessel. This relative movement generates fluid shear acting on the fibres to dislodge or detach cells adhered to the fibres or disposed within the bundle of fibres. In examples, moving the cell culture construct relative to the process vessel comprises reciprocally moving at least one of the cell culture construct and the process vessel. For example, a moving mechanism may be used to move the end support of the cell culture construct relative to at least a part of the process vessel. This movement also acts to move and separate the fibres, increasing detachment and/or dislodgement, allowing cells or cell products to be removed from the process vessel.

In other examples, the cell culture construct may be shaken within the process vessel, and the shaking may cause detachment of the cells from the fibres. The shaking may be in a direction parallel to the fibres, or transverse to the fibres, or a combination of different directions.

In examples, the cell culture construct (and optionally the process vessel) may be centrifuged. In particular, the cell culture construct (and optionally the process vessel) may be held at one end, for example the first vessel end where the end support is disposed, and subject to a centrifugal force. The centrifugal force may encourage cell detachment from the fibres or other structure, and also urge the cells towards one end of the process vessel, which makes collection of the cells easier.

In examples, the harvesting method may include detaching cells from the plurality of fibres. In particular, the cells may be adherent cells that are adhered to internal or external surfaces of the fibres, and the harvesting method may comprise detaching the cells from the fibres. The detachment may be achieved by a fluid flow, as set out above, and/or by use of a cell detachment agent, and/or by applying heat and/or cooling, and/or by applying UV light, and/or by scraping or other physical detachment technique.

In examples, the harvesting method includes collecting detached cells at a lower end of the process vessel, for example by centrifugation or by gravity sedimentation. In examples, the harvesting method may include concentrating the detached cells at the lower end of the harvesting vessel. For example, the process vessel may comprise a narrowed lower portion in which the cells are allowed to accumulate by gravity. Once accumulated, the cells may be removed from the lower portion of the process vessel, and/or fluid may be removed from the volume about the cells. In examples, the cells can be extracted via an outlet port at the lower portion of the process vessel.

In examples, the harvesting method may include extracting a cell product from the process vessel. In particular, in some examples the harvested product is a cell product and not the cultured cells themselves. For example, the harvested product may be a cell product expressed by the cultured cells into the fluid within the process vessel. To harvest such a cell product the harvesting method may include generating a fluid flow through the process vessel, from an inlet to an outlet, to entrain the cell product in the fluid flow. The fluid extracted from the process vessel may be filtered or otherwise processed to remove the cell product from the fluid. In such an example, the harvesting method may be carried out periodically or continuously during the cell culturing method, or after the cell culturing method.

Advantageously, the width of the process vessel may vary between the first vessel end and the second vessel end, and/or be changeable, in order to vary and/or control the flow pattern of the fluid flow during the harvesting method. For example, as described above, the vessel width may increase and/or decrease between the first vessel end and the second vessel end.

In examples, the harvesting method may be intentionally incomplete in that some residual cells are left on the fibres to act as a starter culture for subsequent batches. In examples, the fibres may be intentionally left with residue which can act as an adherent material for subsequent seeding steps.

### Seeding System and Seeding Method

According to a further aspect of the present invention there is also provided a seeding method for seeding cells in the cell culturing system described above. The seeding method comprises generating a fluid flow of a seed cell suspension through the process vessel.

By flowing the seed cell suspension through the process vessel, cells are seeded in the process vessel. Adherent cells would adhere to a support structure in the process vessel, for example on the fibres of the cell culture construct or any other suitable structure present in the cell culture construct, for example scaffolds. The cells and/or the support structure can be modified to enhance adherence, for example by magnetic, chemical or electrical means. Non-adhered cells would be distributed through the process vessel.

In examples, the fluid flow may be in a direction transverse to the plurality of fibres. For example, the fluid flow may be generated at one or more side ports in the process vessel, which direct the fluid flow in a direction transverse to the fibres. Such a fluid flow will convey the seed cells directly towards the fibres, causing cell-fibre interactions that promote adherence. The transverse fluid flow will also move and deform the fibres, further improving dispersion of the seed cells through the process vessel and particularly within the bundle of fibres.

**In** other examples, the fluid flow may be in a direction parallel to the plurality of fibres. For example, the fluid flow may be from the second ends towards the first ends of the plurality of fibres. In other examples, the fluid flow may be from the first ends towards the second ends of the plurality of fibres. Such a flow along the external surfaces of the fibres to promote cell-fibre interactions to encourage cell adherence. The fluid flow will also move and deform the fibres, improving dispersion of the seed cells through the process vessel and particularly within the bundle of fibres.

**In** other examples, the plurality of fibres comprise hollow, open-ended fibres and the fluid flow may be provided through the plurality of fibres. In some examples the cells are provided in lumina of the fibres, in which case the fluid flow through the fibres will position the seed cells in the fibres for culturing. In other examples, providing the fluid flow through the fibres may create a controlled, low shear fluid flow that promotes cell dispersion and/or adhesion.

In examples, the seeding method may include moving the cell culture construct relative to the process vessel. In particular, the seeding method may comprise moving the fibres relative to a fluid in the process vessel. This relative movement provides for improved cell dispersion through the process vessel, and promotes cell-fibre interactions for adherence. In examples, moving the cell culture construct relative to the process vessel comprises reciprocally moving at least one of the cell culture construct and the process vessel. For example, a moving mechanism may be used to move the end support of the cell culture construct relative to at least a part of the process vessel. This movement also acts to move and separate the fibres, increasing the cell dissemination within the process vessel and allowing the seed cells to more easily reach fibres within the bundle of fibres.

In examples, the seed cell suspension comprises adherent cells for adhering to the plurality of fibres. The seed cells may be adhered to internal and/or external surfaces of the fibres.

Advantageously, the width of the process vessel may vary between the first vessel end and the second vessel end, and/or be changeable, in order to vary and/or control the flow pattern of the fluid flow during the seeding method. For example, as described above, the vessel width may increase and/or decrease between the first vessel end and the second vessel end.

In examples, the seeding method may include rotating the cell culture construct either independently or in combination with the process vessel. The process vessel may be rotated at an incline (relative to the direction of the vessel length). The rotation may encourage cell-fibre interactions and dissemination of the cells throughout the process vessel.

In some examples, the process vessel and cells are rotated intermittently. In some examples, the process vessel and cells are rotated at a speed of between 0 to 30 rpm. In some examples, the process vessel and cells are rotated at a speed of about 12 rpm. In some examples, the process vessel and cells are rotated for 1 second to about 30 minutes every 10 seconds to 30 minutes. In some examples, the process vessel and cells are rotated for about 30 seconds every 5 minutes. That is to say that the process vessel is rotated for a period of 30 seconds and then remains stationary for 5 minutes before being rotated again. The process vessel may be rotated for at least 1 hour. For example, at least 1, 2, 3, 4 or 5 hours.

In some examples, the process vessel may be rotated continuously. The process vessel may be continuously rotated at a speed of between 0 and 30 rpm. In some examples, the process vessel is continuously rotated at a speed between 0 and 4 rpm. In some examples, the process vessel is continuously rotated for at least 3 hours. For example, about 3.5 to about 5 hours.

Rotation of the process vessel and cells may be used to exert a centrifugal force on the cells. As such, the centrifugal force may be more than 0.001 N. For example, the centrifugal force may be at least 0.001 N. In some examples, the centrifugal force may be between 0 and 50 N. The application of centrifugal force may help to move the cells through the process vessel module and bring the cells into contact with the fibres. Without being bound by theory, this may help to improve the attachment efficiency of the cells to the fibres. Intermittent rotation of the process vessel may help introduce motive centrifugal forces to free-floating cells which may improve mixing. The increased mixing may help provide an even distribution of cells across fibre surfaces upon contact and attachment.

The force applied by rotation may be affected by the positioning of the process vessel in relation to the axis of rotation. For example, the process vessel may be positioned so as to have an offset between the mid-point of the axial centreline of the process vessel and the axis of rotation. The centreline of the process vessel refers to the plane running axially through the centre of the process vessel longitudinally. The mid-point of the centre line refers to the distance halfway along the centreline. Providing an offset in combination with the rotation described above may help to mix the cells within the process vessel. Without being bound by theory, the mixing imparted by the offset and rotation may increase the surface area of the fibres that is contacted by the cells and may therefore improve the attachment efficiency of the cells to the fibres.

Maintaining the process vessel and/or rotation (intermittent or continuous) may help improve cell attachment and/or increase the number of viable of cells (i.e. viable cells attached to the hollow fibres). For example, increase the attachment efficiency in comparison to methods not including a maintaining step and/or rotation as described herein by at least 5%, 10%, 15%, 20%, 25%, 30%, 35%, 40%, 45%, 50%, 55%, 60%, 65%, 70%, 75%, 80%, 85%, 90%, 95%, 100% or more.

In examples, the process vessel may be inclined at an angle of between 5 degrees and 85 degrees from the horizontal, for example between 5 degrees and 45 degrees from the horizontal, for example between 8 degrees and 40 degrees from the horizontal, for example between 10 degrees and 35 degrees from the horizontal, for example between 15 degrees and 25 degrees from the horizontal, for example about 20 degrees from the horizontal.

### Cell Culture Production Method

According to a further aspect of the present invention, there is provided a cell production method comprising the seeding method, the cell culturing method, and the harvesting seeding method described above.

In examples, the seeding method and/or the cell culturing method, and/or the harvesting method are carried out in the same process vessel or in different process vessels.

In examples, the harvesting method is a method of harvesting the cultured cells and/or a method of harvesting a cell product.

In examples, the method may be a method of producing a cultured food product, and wherein the cells comprise food product cells, for example animal cells or animal cell precursors, such as muscle cells or muscle cell precursors, including fibroblasts, skeletal muscle cells, smooth muscle cells, and/or myoblasts. In other examples, the food product cells may comprise plant cells, or fungal cells, or bacterial cells. In some examples, the food product cells may comprise a mixture of any of these cell types.

In other examples, the method is a method of culturing cells for the production of a biochemical product. In examples, the biochemical product may be the cultured cells themselves, or it may be a cell product expressed by the cultured cells. Such cell products may be proteins, enzymes, primary metabolites, and/or secondary metabolites. The biomedical product may be used for medical purposes, including for biodefence to counter biological threats.

In other examples, the method is a method of culturing cells for the production of a biopharmaceutical product. The biopharmaceutical product may be the cultured cells themselves, fragments or parts of the cultured cells, or a cell product expressed by the cultured cells.

In other examples, the method is a method of culturing cells for the production of a cosmetic, biomass, cell or fermentation product. Such products may be the cultured cells themselves, or cell products.

### Cultured Food Product

In another aspect of the invention there is provided a cultured food product comprising cultured cells, or a product of the cultured cells, obtained by the cell production method described above.

In such examples, the cell culturing system may be a food product cell culturing system, and the cell culture construct may be a food product cell culture construct.

The food product cell culturing system, and the food product cell culture construct may be used to culture adherent cells of any type suitable for food production. In particular, the cultured food product may be a cultured meat product, or a cultured plant product, or a cultured fungal product, or a cultured bacterial product, or a cultured algal product. In other examples, the cultured cells may be further processed into a food product.

A cultured meat product is a meat product comprising fragments of cultured meat. The cultured meat product may include flavouring and other additives. The cultured meat product may be mixed with other sources of protein, such as plant proteins, fungal, bacterial and algal products.

A cultured plant product is a plant product comprising fragments of cultured plant cells or products of the cultured plant cells. The cultured plant product may include flavouring and other additives. The cultured plant product may be mixed with other cultured or non-cultured food products, such as a cultured meat product, a cultured fungal product, a cultured bacterial product, and/or a cultured algal product.

A cultured fungal product is a fungal product comprising fragments of cultured fungal cells or products of the cultured fungal cells. The cultured fungal product may include flavouring and other additives. The cultured fungal product may be mixed with other cultured or non-cultured food products, such as a cultured meat product, a cultured plant product, a cultured bacterial product and/or a cultured algal product.

A cultured bacterial product is a bacterial product comprising fragments of cultured bacterial cells, the bacterial cells themselves, or products of the cultured bacterial cells. The cultured bacterial product may include flavouring and other additives. The cultured bacterial product may be mixed with other cultured or non-cultured food products, such as a cultured meat product, a cultured plant product, a cultured fungal product, and/or a cultured algal product.

A cultured algal product is a algal product comprising fragments of cultured algal cells, the algal cells themselves, or products of the cultured algal cells. The cultured algal product may include flavouring and other additives. The cultured algal product may be mixed with other cultured or non-cultured food products, such as a cultured meat product, a cultured plant product, a cultured fungal product, and/or a cultured bacterial product.

The examples described above relate to a food product cell culturing system having a food product cell culture construct. Such a system, module and construct are for culturing cells to produce a cultured food product.

### Cultured Meat Product

In certain embodiments, the cells and/or cell precursors are derived from at least one comestible animal cell, including animal cells and/or animal cell precursors, for example muscle cells, adipocyte cells and/or cell precursors. The term "meat product" applies generically to products derived from animal cells for the purposes of this application. Optionally, the muscle cells or muscle cell precursors include one or more of fibroblasts, skeletal muscle cells, smooth muscle cells, and/or myoblasts; further optionally wherein the muscle cells are derived from one or more of non-human embryonic stem cells and/or pluripotent stem cells.

The cells seeded onto the fibres may include muscle cells, adipocyte cells and/or cell precursors. Muscle cells include those cells making up contractile tissue of animals or cells that can differentiate into muscle cells. Muscle cells are derived from the mesodermal layer of embryonic germ cells. Mature muscle cells contain contractile filaments that move past each other and change the size of the cell. They are classified as skeletal, cardiac, or smooth muscles. As used herein, the term "cells that can differentiate into muscle cells" and "muscle cell precursors" refers to stem cells and muscle progenitor cells that can differentiate into muscle cells (e.g. mature muscle cells). As used herein, the term "cell precursors" relating to adipose cells refers to mesenchymal stem cells.

Muscle cells may include those cells normally found in muscle tissue, including smooth muscle cells, cardiac muscle cells, skeletal muscle cells (e.g., muscle cells or myocytes, myoblasts, myotubes, etc.), and any combination thereof. Muscle cells may include myoblasts, myotubes, myofibrils, and/or satellite cells.

The cells may further include adipose or fat cells. Adipose or fat cells include any cell or group of cells composed in a fat tissue, including, for example, lipocytes, adipocytes, adipocyte precursors including, pre-adipocytes and mesenchymal stem cells.

The cells may be derived from any source animal. As the bioreactor systems described herein may be for use in making food products, the cells may not be derived from a human. In some examples, the cells may be derived from bovine, ovine, equine, porcine, caprine, avian, fish, insect, crustaceans, cephalopod, mollusc and/or camelid animals. Preferably the cells may be derived from a bovine, porcine, avian and/or ovine animal. For example, the cells may be derived from a cow, pig, chicken, fish, squid, insect, oyster and/or sheep.

### Cultured Plant Product

In certain embodiments, the cells and/or cell precursors are derived from at least one comestible plant cell.

A very wide range of plant cells can be suitable for culturing to form a cultured food product. These can include cells from a plant seed, a leaf, a flower, a scale, an ovary, an ovule, an embryo, a pollen, an adventitious bud, an adventive embryo, an adventitious root a stem, a root, an anther, a filament and a growing point such as a shoot apex, a terminal bud, a lateral bud, a root apex and an axillary bud. Typically, plant callus cultures are especially preferred but any suitable culturing method can be used, including co-culturing.

Suitable plant types include, but are not limited to, onion (*Allium cepa*), garlic (*Allium sativum*)*,* celery (*Apium graveolens*)*,* asparagus (*Asparagus officinalis*), sugar beet (*Beta vulgaris*)*,* cauliflower (*Brassica oleracea* var. *botrytis*)*,* brussels sprout (*Brassica oleracea* var. *gemmifera*), cabbage (*Brassica oleracea* var. *capitata*), rape (*Brassica napus*)*,* caraway (*Carum carvi*)*,* coptis rhizome (*Coptis japonica*)*,* chicory (*Cichorium intybus*)*,* summer squash (*Curcurbita pepo*)*,* carrot *(Daucus carota*)*,* carnation (*Dianthus caryophyllus*)*,* buckwheat (*Fagopyrum esculentum*)*,* fennel (*Foeniculum vulgare*)*,* strawberry (*Fragaria chiloensis*)*,* soybean (*Glycine max*)*,* sweet potato (*Ipomoea batatas*)*,* lettuce (*Lactuca sativa*)*,* tomato (*Lycopersicon esculentum*)*,* alfalfa *(Medicago sativa*)*,* tobacco (*Nicotiana tabacum*)*,* rice (*Oryza safiva*)*,* parsley *(*P*etroselinum hortense*)*,* pea (*Pisum sativum*)*,* egg plant (*Solanum melongena*)*,* faba bean (*Vicia faba*)*,* potato (*Solanum tuberosum*)*,* wheat (*Triticum aestivum*)*,* maize (*Zea mays*)*,* sunflower (*Helianthus annuus*)*,* foliage plants such as clovers, for example white clover (*Trefolium repens*)*,* snapdragon *(Antirrhinum majus*)*,* mouse-ear cress (*Arabidopsis thaliana*)*,* dandelion (*Taraxacum officinale*), cymbidium (*Cymbidium*) and the like; trees such as citrus (for example *Citrus trifoliata*) and other citrus, common coffee (*Coffea arabica*), cacao (*Theobroma cacao*)*,* ribbon gum *(Eucalyptus*)*,* para rubber tree (*Hevea brasiliensis*)*,* almond *(Prunus amygdalus*), Norway spruce (*Picea abies*), pine genus (*Pinus*)*,* apple (*Malus pumila*), apricot (*Prunus armeniaca*)*,* persimmon (*Diospyros kaki*)*,* fig (*Ficus carica*), chestnut (*Castanea crenata*) and the like. *Theobroma cacao* is of particular interest.

In various examples, any suitable culturing method can be used. Examples thereof for plants include dedifferentiation (callusing) and redifferentiation of plant tissue, anther culture, shoot apex culture, protoplast culture, batch culture, co-culture, clonal cell culture, seed culture, an ovary culture method, an ovule culture method, an embryo culture method and a pollen culture method.

A particular advantage for culturing plant cells in a hollow fibre reactor is that some plant cells are particularly susceptible to shear damage. This is problematic for bioreactors such as stirred tank reactors, especially at larger scale. A hollow fibre bioreactor is well suited to provide low shear environments due to the shear-independent perfusion of nutrients/waste products through the membranes.

A further advantage is that plant cells or fungal cells do not typically adhere strongly to the fibres typically used in hollow-fibre reactors and hence a grown cell mass can be more easily removed for harvesting. This is especially useful for larger-scale cell masses grown as foodstuffs. Plant cells are typically different to the animal muscle cells typically used in cultured / cultivated meat in that they can grow when not adhered to a suitable surface. Hence the factors in fibre bundle design relating to seeding of cells onto the fibres do not usually apply for plant cells. However, factors of fibre spacing to control the gaps between fibres and allow diffusion of nutrients etc and maximising the space available for cell growth apply. The diffusion of nutrients and waste products through the membranes depends (amongst other factors) on the external surface area of the fibres. Hence the rationale of controlling the fibre density to simultaneously maximise the available surface area (this time to enhance diffusion) and available volume for cell growth still applies to non-mammalian cell types.

### Cultured Fungal Product

In certain embodiments, the cells and/or cell precursors are derived from at least one comestible fungal cell.

Fungal cells, such as mushroom, moulds or yeasts, can also be cultured in a hollow fibre reactor to form a comestible cultured food product. These can include mushroom types such as enoki mushroom (*Flammlina velutipes*), shiitake mushroom (*Lentinula edodes*)*,* bunashimeji mushroom (*Hypsizygus marmoreus*)*,* fried chicken mushroom (*Lyophyllum decastes*)*,* horse mushroom (*Agaricus arvensis*), puffball (*Lycoperdon gemmatum*)*,* mannentake mushroom (*Ganoderma lucidum*)*,* oyster mushroom (*Pleurotus ostreatus*)*,* maitake mushroom (*Grifola frondosa*), matsutake mushroom (*Tricholoma matsutake*) as well as yeasts such as Bakers Yeast (*Saccharomyces cerevisiae*)*.* The low adhesion of filamentous cell masses to the fibres of a hollow fibre reactor typically enhances harvesting and removal of the cultured fungal product from the bioreactor. Yeasts, fungi, algae and bacteria used in the culturing methods may be modified, for example by genetic modification techniques.

### Cultured Bacterial Product

In certain embodiments, the cells and/or cell precursors are derived from at least one comestible bacterial cell.

Bacterial cells can also be cultured in hollow fibre reactors to form a comestible cultured food product. Suitable bacteria can include photosynthetic bacteria such as *Rhodospillum molischianum,* nitrogen-fixing bacteria such as *Azotobacter chroococcum,* acetic acid bacteria including *Acetobacter aceti,* butyric acid fermenting bacteria such as *Clostridium butyrium,* acetone-butanol fermenting bacteria such as *Clostridium acetobutylicum,* halophilic bacteria including *Haloarcula japonica,* cryophilic bacteria including *Colwellia psychrerythraea,* acidophilic bacteria including *Sulfolobus tokodaii* and alkaliphilic bacteria, for example *Bacillus alcalophilu. Bacteria used in the culturing methods may be modified, for example by genetic modification techniques.*

### Cultured Algal Product

In certain embodiments, the cells and/or cell precursors are derived from at least one comestible algal cell.

Examples of algal cells for culturing a comestible cultured food product include spirulina, chlorella, sea moss, red algae, brown algae, blue-green algae, macroalgae, and microalgae. Algal cells may include one or more of chlorophyta, rhodophyta, glaucophyta, chlorarachniophytes, euglenids, heterokonts, dinoflagellates, haptophyta, cryptomonads. Algal cells used for culturing may be modified, e.g., by genetic modification techniques.

### Biochemical and Biopharmaceutical Product

Biochemical products are chemical products produced by biological means. For example, butanol produced by fermentation is an example of a biochemical product. Biopharmaceutical products are products produced by biological means that have a medical or pharmaceutical use. Biochemical products may be biopharmaceutical products and vice versa.

In examples, the cell culturing system and methods described above may be used for culturing cells for the production of biochemical products such as alcohols, organic acids, fatty acids/fatty acid salts, aldehydes/ketones and polyhydroxyalkanoates. In examples, the cell culturing system and methods described above may be used for culturing cells for the production of biopharmaceutical products such as recombinant proteins, antibodies, viral particles, vaccines, organoids and tissues. For example, the apparatus and methods described above may be used for culturing cells suitable for production of recombinant proteins such as mammalian cells (such as Chinese Hamster Overy (CHO) cells, Human Embryonic Kidney (HEK) cells (e.g. HEK293), baby hamster kidney (BHK-21) cells, or myeloma cells), bacterial cells (such as *Escherichia coli* cells, ), yeast cells (such as *Saccharomyces cerevisiae, Pichia pastoris, Hansenula polymorpha, Yarrowia lipolytica , Kluyveromyces lactis, Schizosaccharomyces pombe* or *Arxula adeninivorans* cells), fungi cells (such as *Aspergillus niger, Aspergillus oryzae, Trichoderma reesei,* or *Chrysosporium lucknowense),* and insect cells (such as the Sf9 cell/baculovirus expression system). Such cells can also be used for the production of viruses and virus like particles which may be used for applications such as vaccine production. Such cells may also be used to produce antibodies, in particular CHO cells, HEK cells, Mouse myeloma NSO cells, E.Coli cells and yeast cells. Antibodies, such as monoclonal antibodies may also be produced by using the apparatus and methods described above to culture hybridoma cells, for example by culturing myeloma cells fused with antibody-producing splenocytes (e.g. B cells). The antibody-producing splenocytes may be derived a mammal such as a mouse. In some examples, the apparatus and methods described above are used for production of organoids or tissue. In such cases, the apparatus and methods described above may be used to culture primary cell types which are obtained directly from living tissue (such as, but not limited to embryonic stem cells, hematopoietic stem cells, neural stem cells, multipotent stem cells, endothelial cells, epithelial cells, mesenchymal stem cells, hepatocytes, melanocytes, and keratinocytes). The apparatus and methods described above may also be used to culture induced stem cells such as induced pluripotent stem cells. These cells may be cultured under suitable conditions to induce differentiation into specific tissues or to produce organoids. Furthermore, the apparatus and methods described above may be used to culture tissue or tissue specific cells such as connective tissue, epithelial tissue, muscle tissue, and nervous tissue cells. Yeasts, bacteria and other cell types used in the culturing methods may be modified, for example by genetic modification techniques.

The cultured cells may express cell products into the surrounding media. In this case, it is possible for these cell products to be filtered or otherwise separated from the media during and/or after culturing.

### BRIEF DESCRIPTION OF THE DRAWINGS

To easily identify the discussion of any particular element or act, the most significant digit or digits in a reference number refer to the figure number in which that element is first introduced.
FIG. 1 illustrates a first example cell culturing system.
FIG. 2 illustrates a second example cell culturing system.
FIG. 3 illustrates a third example cell culturing system.
FIG. 4 illustrates a fourth example cell culturing system.
FIG. 5 illustrates a fifth example cell culturing system.
FIG. 6 illustrates an example cell culturing system with a plurality of cell culture constructs.
FIG. 7A illustrates an example arrangement of fibres in a cell culture construct.
FIG. 7B illustrates an alternative example arrangement of fibres in a cell culture construct.
FIG. 8A illustrates a further alternative example arrangement of fibres in a cell culture construct.
FIG. 8B illustrates a further alternative example arrangement of fibres in a cell culture construct.
FIG. 9 illustrates an example process vessel of a cell culturing system.
FIG. 10 illustrates an alternative example process vessel of a cell culturing system.
FIG. 11A and FIG. 11B illustrate an example process vessel of a cell culturing system, where the process vessel can change shape.
FIG. 12 illustrates an example moving mechanism of a cell culturing system.
FIG. 13 illustrates a further example moving mechanism of a cell culturing system.
FIG. 14 illustrates an example end support of a cell culture construct.

### DETAILED DESCRIPTION

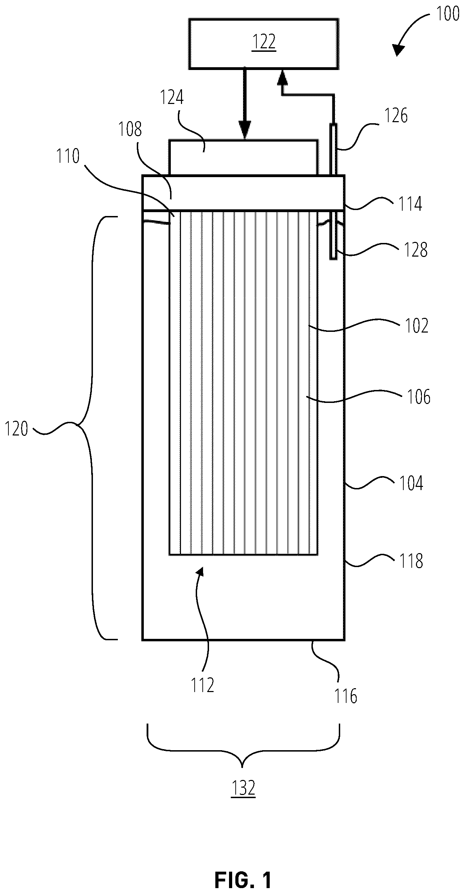

FIG. 1 to FIG. 5 show different example cell culturing systems 100, 200, 300, 400, 500. Common features maintain the same reference numerals through FIG. 1 to FIG. 5. The cell culturing system 100, 200, 300, 400, 500 may be termed a cell culturing bioreactor, or bioreactor module.

In each example, the cell culturing system 100, 200, 300, 400, 500 includes a process vessel 104 in which a cell culture construct 102 is received. The cell culture construct 102 comprises an end support 108 and a plurality of hollow fibres 106. First ends 110 of the fibres 106 are supported by the end support 108, for example embedded therein. The fibres 106 extend into the process vessel 104 and second ends 112 of the fibres 106 are free to move within the process vessel 104.

In these examples the fibres 106 are hollow, open-ended fibres. The first ends 110 of the fibres 106 are accessible at the end support 108 for fluid communication with lumen of the fibres 106 from external of the end support 108.

The process vessel 104 includes a first vessel end 114, a second vessel end 116, and a vessel side wall 118 extending between the first vessel end 114 and the second vessel end 116. The process vessel 104 has a vessel length 120 between the first vessel end 114 and the second vessel end 116, and a vessel width 132. In these examples, the process vessel 104 has a uniform cross-section along the length of the process vessel 104. In particular, the process vessel 104 has a constant vessel width 132. The process vessel 104 may have a square, rectangular, or circular cross-section, for example.

The cell culturing system 100, 200, 300, 400, 500 includes a fluid flow system 122. The fluid flow system 122 is connected to the process vessel 104 for generating a fluid flow through the cell culturing system 100, 200, 300, 400, 500, as described further below. The fluid flow system 122 may include one or more pumps, valves and sensors for controlling the fluid flow rate into/through the process vessel 104, and into/through the cell culture construct 102.

In particular, the fluid flow system 122 may be attached to the process vessel 104 and/or the fibres 106 by an inlet port and/or a second port. The inlet port and the outlet port may each include a threaded male or female type connector, for example, BSP or NPT, or a hygienic specification, for example, Tri-Clamp, Ingold, Luer, Hansen-type, or DIN11851. The inlet port and the outlet port may each have inner walls that are substantially parallel, curved or tapered. A tapered design for an connector may create a funnel such that the collection of suspended solids, for example cells, are collected more effectively than a straight walled exit port. The inlet port and the outlet port may be arranged relative to the process vessel 104 such that the are parallel to the end support 108, or angled with respect to the end support 108.

In some examples, the process vessel 104 is made of fully welded stainless steel in which the welds are suitably polished and internal surfaces smoothed. That can help to reduce harbouring of microbiological contaminants.

In preferred examples the cell culturing system 100, 200, 300, 400, 500 includes a base unit that holds the process vessel 104. The process vessel 104 may be detachable from the base unit and the fluid flow system 122. The process vessel 104 may be held in a vertical, horizontal, or sloped position.

The process vessel 104 may be similar to a conventional tubular membrane housing used in filtration applications. As described above, the process vessel 104 may comprise one or more fluid entry or exit ports enabling fluid to pass in and out of the extra-capillary space within the process vessel 104, external to the fibres of the cell culture construct 102.

The process vessel 104 may be substantially similar to a glass or stainless steel bioreactor vessel. The process vessel 104 may be an upright vessel that is freestanding, or mounted on a frame (base). The process vessel 104 has an internal height to width aspect ratio of between 0.5 and 5.0. The process vessel 104 may have a base or top which is conical, dished or flat. The cell culturing system 100, in particular the process vessel 104, may include a jacket surrounding the vessel side wall 118 of the process vessel 104, and the jacket may enable temperature maintenance, heating or cooling of the process vessel 104 by passing fluids such as steam, water or oil through the jacket (in between the jacket and the walls of the process vessel 104). The cell culturing system 100 may include an incubator, an environmental chamber, or a water bath in which the process vessel 104 may be placed during use. This can help to control temperature and other environmental aspects during food product culturing. The process vessel 104 may be hermitically sealed by the end support 108 (and possible additional endplates / head plates), allowing the internal pressure of the process vessel 104 to be controlled, for example elevated. This can also maintain the sterility of the internal space of the process vessel 104 to avoid microbiological or other contaminant ingress into the process vessel 104 during operation. The process vessel 104, in particular the side walls and/or the end support 108 and/or any additional endplate/headplate, may include one or more ports that allow sensors and/or gauges to be connected. Sensors and/or gauges may be provided to detect pressure, temperature, . Sensors may include one or more of pressure sensors, temperature sensors (e.g., thermowells), pH sensors, foam sensors, level sensors (e.g., point level switches), dissolved oxygen (dO) sensors, capacitance sensors, gas analysers, or optical density sensors. The process vessel 104, in particular the side walls and/or the end support 108 and/or any additional endplate/headplate, may include one or more ports that allow equipment to be connected. For example, fixed or rotating spray heads (e.g., spray balls) for cleaning, bursting discs, pressure relief valves, overflow pipes, condensers, agitator shafts, sight glasses, lights, addition tubes, or dip tubes.

In some examples, the process vessel 104 may be a modified bioreactor enabling retrofitting of the cell culture construct 102.

The end support 108 may comprise a sealing material, for example a resin, glue, or potting material. The sealing material can be introduced between the fibres 106 in a fluid form, for example using a centrifuge, and then cured to form the end support 108. After curing an end of the end support 108 may be cut off to expose ends of the fibres and their lumina. In some examples, the end support 108 comprises a cup (e.g., a tube) within which the fibres are sealed.

In the example cell culturing system 100 of FIG. 1 the fluid flow system 122 is connected to a first port 124 (an inlet port) for inputting a fluid flow into the process vessel 104, and to a second port 126 (outlet port) for outputting a fluid flow from the process vessel 104.

The first port 124 is connected to the end support 108 and provides an input fluid flow to the lumina of the fibres 106. In this way, fluid is input into the process vessel 104 through the lumina of the fibres 106. The second port 126 is located at the first vessel end 114, and in particular comprises a tube 128 that extends through the end support 108 and into the process vessel 104 for output of fluid flow from the process vessel 104.

In this way, the fluid flow system 122 can generate a fluid flow through the process vessel 104. In some examples the fibres 106 are porous, and fluid may pass through the walls of the fibres 106 as the fluid flows from the first ends 110 to the second ends 112. Fluid may also pass through the length of the fibres 106 are exit at the second ends 112 into the process vessel 104.

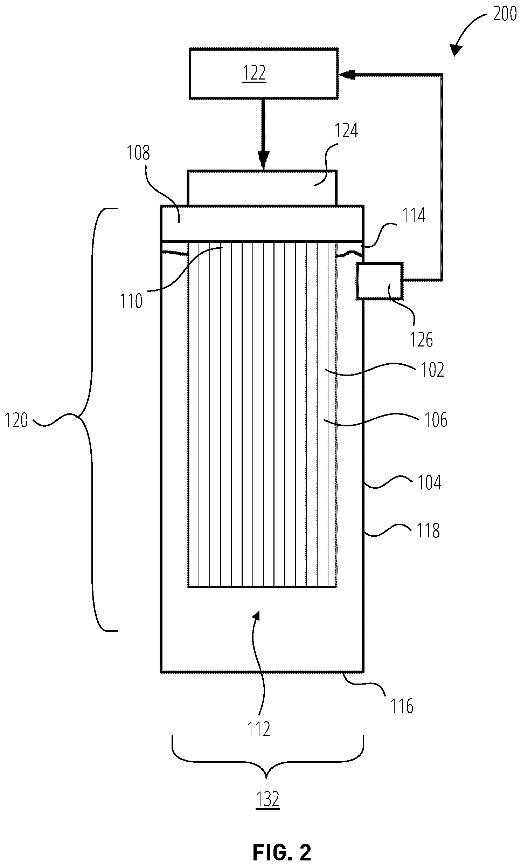

In the example cell culturing system 200 of FIG. 2, the fluid flow system 122 is connected to a first port 124 (inlet port) for inputting a fluid flow into the process vessel 104, and to a second port 126 (outlet port) for outputting a fluid flow from the process vessel 104.

As with the example of FIG. 1, the first port 124 is connected to the end support 108 and provides the input fluid flow to the lumina of the fibres 106. In this way, fluid is input into the process vessel 104 through the lumina of the fibres 106.

In this example, the second port 126 is located at the first vessel end 114, and in particular at a position on the vessel side wall 118 proximate to the first vessel end 114. The second port 126 comprises an opening into the process vessel 104 and a connector for connecting a tube or the like, as discussed above.

In this way, the fluid flow system 122 can generate a fluid flow through the process vessel 104. In some examples the fibres 106 are porous, and fluid may pass through the walls of the fibres 106 as the fluid flows from the first ends 110 to the second ends 112. Fluid may also pass through the length of the fibres 106 are exit at the second ends 112 into the process vessel 104.

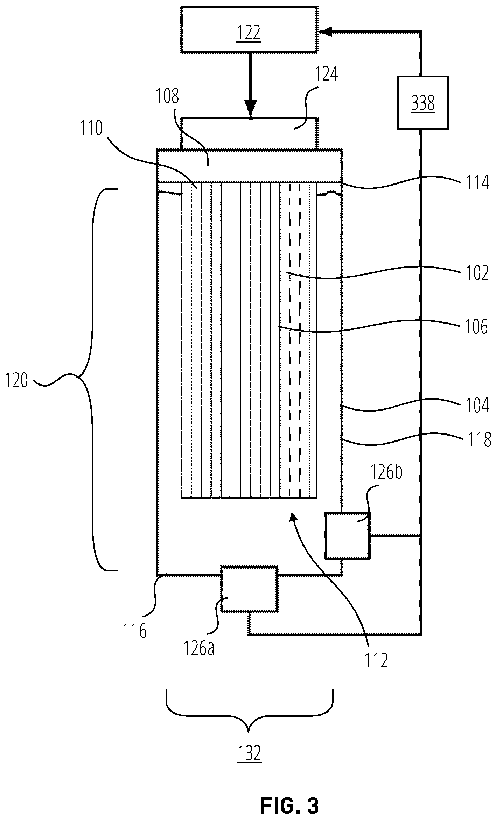

In the example cell culturing system 300 of FIG. 3, the fluid flow system 122 is connected to a first port 124 (inlet port) for inputting a fluid flow into the process vessel 104, and to a second port 126a, 126b (outlet port) for outputting a fluid flow from the process vessel 104.

As with the examples of FIG. 1 and FIG. 2, the first port 124 is connected to the end support 108 and provides the input fluid flow to the lumina of the fibres 106. In this way, fluid is input into the process vessel 104 through the lumina of the fibres 106.

In this example, the second port 126 is located at the second vessel end 116. In some examples, the second port 126a is located in the second vessel end 116 and/or the second port 126b is located in the vessel side wall 118. In these examples the second port 126 is located at or near the second vessel end 116. The outlet port 126a, 126b comprises an opening into the process vessel 104 and a connector for connecting a tube or the like, as discussed above.

In this way, the fluid flow system 122 can generate a fluid flow through the process vessel 104. In some examples the fibres 106 are porous, and fluid may pass through the walls of the fibres 106 as the fluid flows from the first ends 110 to the second ends 112. Fluid may also pass through the length of the fibres 106 are exit at the second ends 112 into the process vessel 104.

As illustrated, a filter 338 may be provided to filter fluid being removed from the process vessel 104. In some examples, the filter 338 may be used to extract a cell product from the fluid flow, the cell product being the desired product to harvest. For example, the cell product may be expressed into the fluid in the process vessel 104 during cell culturing.

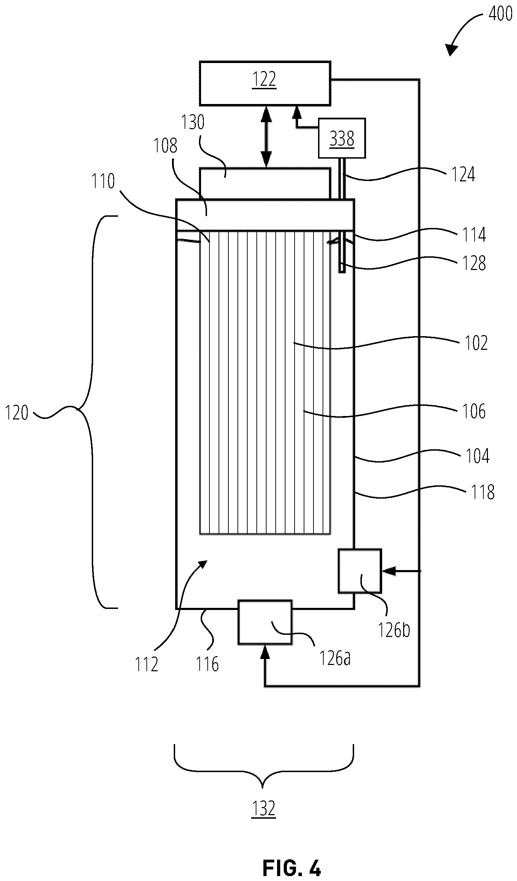

The example cell culturing system 400 of FIG. 4 is similar to the cell culturing system 300 of FIG. 3, except that the flow direction is reversed. In particular, in this example the first port 124 located at the first vessel end 114, and the second port 126 is located at the second vessel end 116. The second port 126 is used as an inlet port and the first port 124 is used as an outlet port. As shown, the second port 126a may be located in the second vessel end 116, or the second port 126b may be located in the vessel side wall 118, proximate to the second vessel end 116. The first port 124 comprises a tube 128 that extends through the end support 108 and into the process vessel 104 for output of fluid flow from the process vessel 104. The fluid flow system 122 may additionally be connected to a third port 130 formed in the end support 108. The third port 130 may be used as an inlet port or as an outlet port. In this way, fluid can be input into the process vessel 104 at the second vessel end 116 and/or through the lumina of the fibres 106, and output through the through first port 124 at the first vessel end 114 and/or through the lumina of the fibres 106.

In some examples the fibres 106 are porous, and fluid may pass through the walls of the fibres 106 as the fluid flows from the first ends 110 to the second ends 112 or vice versa.

As illustrated, a filter 338 may be provided to filter fluid being removed from the process vessel 104. In some examples, the filter 338 may be used to extract a cell product from the fluid flow, the cell product being the desired product to harvest. For example, the cell product may be expressed into the fluid in the process vessel 104 during cell culturing.

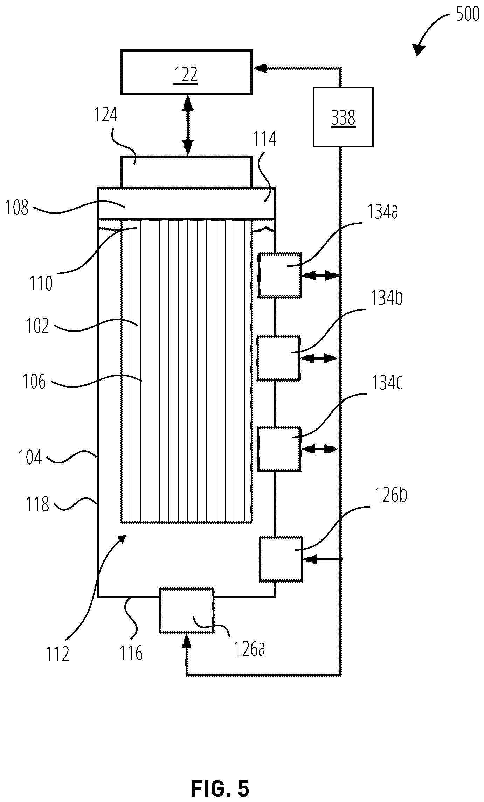

In the example of FIG. 5 the cell culturing system 500 further comprises one or more side ports 134, in this example three side ports 134a, 134b, 134c. These are in addition to the first port 124, which is this example is formed at the first vessel end 114, and the second port 126, which in this example is formed at the second vessel end 116. As described above, the second port 126 may be a second port 126a formed in the second vessel end 116 or the second port 126b may be formed in the vessel side wall 118 proximate to the second vessel end 116. The first port 124, second port 126 and side ports 134 may be used as inlet ports and/or as outlet ports to generate different fluid flows through the process vessel 104.

In particular, in each example, fluid flow through the first port 124 and the second port 126 may generate fluid flow in a direction generally parallel to the fibres 106, in the longitudinal direction of the process vessel 104. This flow may be in one direction, or reversible. The flow may be constant, or intermittent, or may have variable flow rate. The side ports 134 can be used to generate a fluid flow in a transverse direction, across the fibres 106.

As illustrated, a filter 338 may be provided to filter fluid being removed from the process vessel 104. In some examples, the filter 338 may be used to extract a cell product from the fluid flow, the cell product being the desired product to harvest. For example, the cell product may be expressed into the fluid in the process vessel 104 during cell culturing.

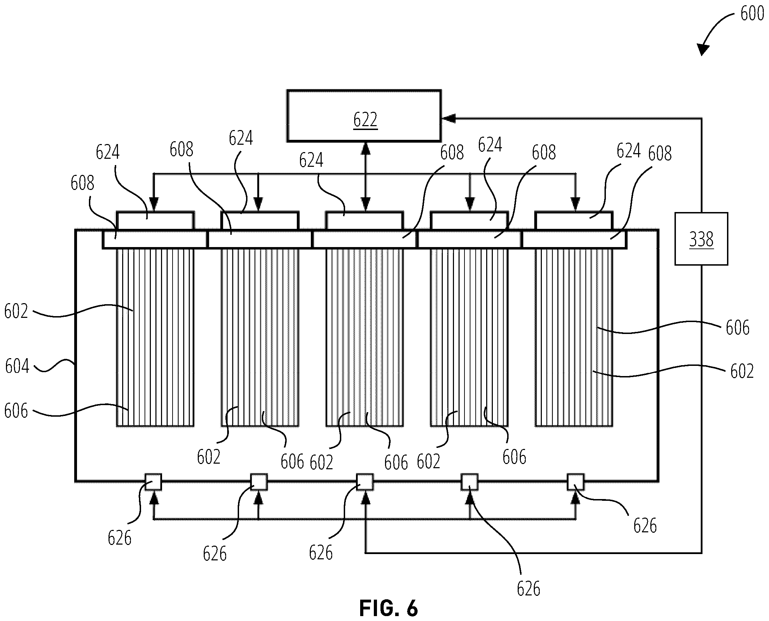

In the example of FIG. 6 the cell culturing system 600 comprises a process vessel 604 in which a plurality of cell culture constructs 602 are received. Each cell culture construct 602 includes an end support 608 and a plurality of fibres 606 extending therefrom into the process vessel 604. As with the above examples, first ends of the fibres 606 are supported in the end support 608 and second ends of the fibres 606 are free to move within the process vessel 604.

The cell culturing system 600 also includes a fluid flow system 622, which is connected to the cell culture constructs 602 and/or to the process vessel 604 by first ports 624 and second ports 626, which may be configured as described with reference to any of FIG. 1 to FIG. 5. In the illustrated example, the first ports 624 are connected to the end supports 608 of the cell culture constructs 602, and the second port 626 are located in the second vessel end, opposite to the end supports 608. It will be appreciated that a tube may be provided, as described with reference to FIG. 1, and/or side ports may be provided, as described with reference to FIG. 5.

Advantageously, the cell culturing system 600 provides for larger scale cell culturing because multiple cell culture constructs 602 are received in a single process vessel 604.

As illustrated, a filter 338 may be provided to filter fluid being removed from the process vessel 604. In some examples, the filter 338 may be used to extract a cell product from the fluid flow, the cell product being the desired product to harvest. For example, the cell product may be expressed into the fluid in the process vessel 604 during cell culturing.

In the examples of FIG. 1 to FIG. 6, the fibres 106, 606 may be hollow, open-ended fibres. The fluid flow system 122, 622 may be connected to lumina of the fibres 106, 606 for generating a fluid flow through the fibres 106, 606, either from the first ends 110 to the second ends 112 or vice versa. In such examples, cells are cultured within the lumina of the fibres 106, 606, on external surfaces of the fibres 106, 606, and/or in the extracapillary space surrounding the fibres 106, 606 within the process vessel 104, 604. Cells may be adherent or non-adherent. Adherent cells may adhere to the fibres 106, 606 or other structures provided in the cell culturing system 100, 200, 300, 400, 500, 600, for example a carrier structure or a scaffold structure.

In other examples, specifically with the cell culturing system 400 of FIG. 4, the cell culturing system 500 of FIG. 5, and the cell culturing system 600 of FIG. 6, the fibres 106, 606 may be solid fibres. In such examples, the fluid flow system 122, 622 generates a fluid flow through the extracapillary space within the process vessel 104, 604, around the fibres 106, 606. In these examples, cells are cultured on the external surfaces of the fibres 106, 606 and/or in the extracapillary space surrounding the fibres 106, 606 within the process vessel 104, 604. Cells may be adherent or non-adherent. Adherent cells may adhere to the fibres 106, 606 or other structures provided in the cell culturing system 100, 200, 300, 400, 500, 600, for example a carrier structure or a scaffold structure.

As explained further below, the cell culturing systems 100, 200, 300, 400, 500, 600 described above may be used for seeding, cell culturing, and/or harvesting of cells. One or more of seeding, cell culturing and harvesting may be performed in the cell culturing system 100, 200, 300, 400, 500, 600.

When used for seeding, a seed cell suspension is flowed into the process vessel 104, 604. The seed cell suspension may be provided to the process vessel 104, 604 by the fluid flow system 122, 622.

In one example, the seed cell suspension may be provided through the second port 126, 626 to directly enter the space within the process vessel 104, 604 around the fibres 106, 606. Fluid may then be extracted from the first port 124, 624. In this way, the seed cell suspension flows over the external surfaces of the fibres 106, 606. The seed cell suspension may comprise adherent cells that adhere to the fibres 106, 606, or may comprise non-adherent cells that are seeded into the space surrounding the fibres 106, 606 within the process vessel 104, 604.

In some examples, the seed cell suspension is flowed through the process vessel 104, 604 in a direction from the second vessel end 116 towards the first vessel end 114. In particular, the seed cell suspension may be input into the process vessel 104, 604 at the second port 126, 626 located at the second vessel end 116, and fluid can be extracted from the first port 124, 624 at the first vessel end 114. This would generate a flow of seed cell suspension moving from the second ends 112 of the fibres 106, 606 towards the first ends 110 of the fibres 106, 606. Such a fluid flow may cause the fibres 106, 606 to spread out, because the second ends 112 are free to move. This will increase the interactions and contacts between the seed cell suspension and the fibres 106, 606, improving seeding of adherent cells onto the fibres 106, 606.

In addition, by extracting fluid via the lumina of the fibres 106, 606 during seeding, a suction effect may be generated that pulls cells onto the external surfaces of the fibres 106, 606, improving adhesion thereto.

In another example, the seed cell suspension is input into the process vessel 104, 604 via the fibres 106, 606, and in particular via the first port 124, 624 connected to the lumina of the fibres 106, 606. The seed cell suspension then exits the fibres 106, 606 at the second ends 112, and can be extracted from the process vessel 104, 604 at the second port 126, 626. In this way, cells may be seeded onto internal surfaces of the fibres 106, 606. Alternatively, non-adherent cells may pass into the process vessel 104, 604 via the fibres 106, 606 for culturing in the space surrounding the fibres 106, 606 within the process vessel 104, 604.

In examples, the seed cell suspension may be input into the process vessel 104, 604 via the lumina of the fibres 106, 606. In such examples, the fibres 106, 606 may provide a slow rate of fluid flow into and through the process vessel 104, 604, which may reduce fluid shear forces acting on the cells and improve adhesion of adherent cells to the fibres 106, 606.

In some examples the seed cell suspension may be input and/or output from the process vessel 104, 604 via the side ports 134, such as those described with reference to FIG. 5. Use of the side ports 134 may generate a flow that is transverse to the fibres 106, 606 and thereby increase the contacts and interactions of the cells and the fibres 106, 606, improving seeding.

After seeding, the cell culturing systems 100, 200, 300, 400, 500, 600 described above may be used for cell culturing. In particular, during cell culturing a cell culture medium may be flowed through the process vessel 104, 604 to provide nutrients and the like to the cells to encourage culturing. In examples, the cell culture medium may be input into the process vessel 104, 604 through the lumina of the fibres 106, 606 via the first port 124, 624, or directly into the space surrounding the fibres 106, 606 through the second port 126, 626. The cell culture medium can be extracted through the other of the first port 124, 624 and the second port 126, 626 to generate a fluid circulation through the process vessel 104, 604.

In examples, the cell culture medium may be input to, or output from, the process vessel 104, 604 via the lumina of the fibres 106, 606. In such examples, the fibres 106, 606 may provide a slow rate of fluid flow through the process vessel 104, 604, which may reduce fluid shear forces acting on the cells to reduce dislodgement of the cells from the fibres 106, 606 or other locations in the process vessel 104, 604 during culturing. Such a fluid flow may also improve nutrient supply to the cells. In particular, cells adhered to the fibres 106, 606 (internally or externally) or would be provided with nutrients from the cell culture medium flowing through the fibres 106, 606 and from the cell culture medium in the space surrounding the fibres 106, 606 within the process vessel 104, 606.

In some examples, the cell culture medium is flowed through the process vessel 104, 604 in a direction from the second vessel end 116 towards the first vessel end 114. In particular, the cell culture medium may be input into the process vessel 104, 604 at the second port 126, 626 located at the second vessel end 116, and fluid can be extracted from the first port 124, 624 at the first vessel end 114. This would generate a flow of cell culture medium moving from the second ends 112 of the fibres 106, 606 towards the first ends 110 of the fibres 106, 606. Such a fluid flow may cause the fibres 106, 606 to spread out, because the second ends 112 are free to move. This will increase the interactions and contacts between the cell culture medium and the fibres 106, 606, improving the supply of nutrients to the cells on or in the fibres 106, 606.

When the cell culturing system 100, 200, 300, 400, 500, 600 is used in cell culturing the cell culturing system 100, 200, 300, 400, 500, 600 may be a perfusion system. In some examples, the fluid flow system 122, 622 may include means of in-situ sterilisation during operation. For example, the cell culture medium may be passed through a UV-light sterilisation step prior to being passed through the bioreactor.

When used in harvesting, the fluid flow system 122, 622 may circulate a fluid through the process vessel 104, 604 and/or cell culture construct 102, 602 to cause detachment of the cells from the fibres 106, 606. The detached cells can then be removed from the process vessel 104, 604, for example by being entrained in a flow that carries them out of the process vessel 104, 604, for example via one of the first port 124 and the second port 126. Further details of various harvesting techniques are provided hereinafter.

As described above, during seeding, cell culturing and/or harvesting the first port 124, second port 126, third port 130 and/or side ports 134 may be used to add, flow or circulate fluid through the cell culture construct 102, 602 (through the fibres 106, 606) and/or through the process vessel 104, 604 around and over the cell culture construct 102, 602. Such fluids may include a seed cell suspension, various cell culture media (growth media), buffer solution(s), cleaning fluids, enzyme-containing solutions, antifoam, steam or chemical reagents, for example, for modifying fluid pH.

As described above, the or each cell culture construct 102, 602 comprises an end support 108, 608 and a plurality of fibres 106, 606 extending from the end support 108, 608. First ends 110 of the fibres 106, 606 are supported in the end support 108, 608, for example embedded therein.

In examples, the or each end support 108, 608 comprises a sealing material, which may be a resin or glue or potting material. The sealing material can be introduced between the fibres 106, 606 in a fluid form, for example by a centrifuge, and then cured to form the end support 108, 608. After curing an end of the end support may be cut off to expose the ends of the fibres 106, 606 and permit fluid access to the lumina of the fibres 106, 606.

In other examples, the or each end support 108, 608 may clamp or otherwise hold the fibres 106, 606, without a sealing material. For example, the fibres 106, 606 may be pushed through openings formed in the end support 108, 608, or pushed through a soft ductile material, or clamped or crimped within a sleeve.

The first ends 110 of the fibres 106, 606 are thereby held in a fibre distribution at the end support 108, 608. The fibre distribution is fixed at the end support 108, 608, but is changeable along the length of the fibres 106, 606 because the second ends 112 are free to move within the process vessel 104, 604.

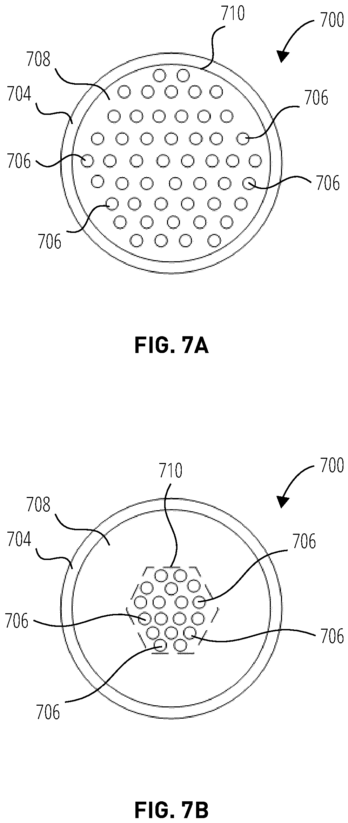

FIG. 7A and FIG. 7B show cell culturing systems 700 with example fibre distributions. In particular, FIG. 7A and FIG. 7B show an end view of a cell culturing system 700, with the cell culture construct 702, in particular the end support 708, received in the process vessel 704. A plurality of fibres 706 are supported in the end support 708.

As illustrated in FIG. 7A and FIG. 7B, the fibre distribution of the fibres 706 within the end support 708 is defined within an envelope 710. In the example of FIG. 7A the envelope 710 is substantially the same size as the end support 708 and the process vessel 704. In the example of FIG. 7B the envelope 710 is smaller than the end support 708 and the process vessel 704, with the fibres 706 being concentrated towards a centre of the end support 708. The example of FIG. 7B provides for increased space between the fibres 706 and the side wall of the process vessel 704 (or an adjacent cell culturing system 700) during use, providing for greater movement of the second ends of the fibres 706.

In the examples of FIG. 7A and FIG. 7B the fibres 706 have a regular fibre distribution within the envelope 710. That is, the fibres 706 are regularly distributed and evenly spaced from each other within the envelope 710, defining a constant fibre density across the envelope 710. However, in other examples, the fibres 706 may have a fibre distribution with varying fibre density across the envelope 710. For example, the fibre density may be greater towards a centre of the envelope 710 than towards an edge of the envelope 710 (i.e., the fibre spacing may be less towards a centre of the envelope 710 than towards an edge of the envelope 710).

In examples, the fibre density of the fibres 706 may be between about 50 fibres/cm² and about 330 fibres/cm².

In the examples of FIG. 7A and FIG. 7B the envelopes 710 are circular or nearly circular (e.g., hexagonal or the like). The end supports 708 have a same or similar shape to the envelopes 710. However, in other examples the end supports 708 and/or the envelopes 710 may be non-circular, for example triangular, square, or rectangular. Such shapes may allow the end supports 708 to be more efficiently arranged when several are received in a process vessel, and may also improve fluid access within the bundle of fibres during seeding, culturing and harvesting. Non-circular end supports 708 and envelopes 710 may also make it easier to remove cultured cells from the fibres after culturing.

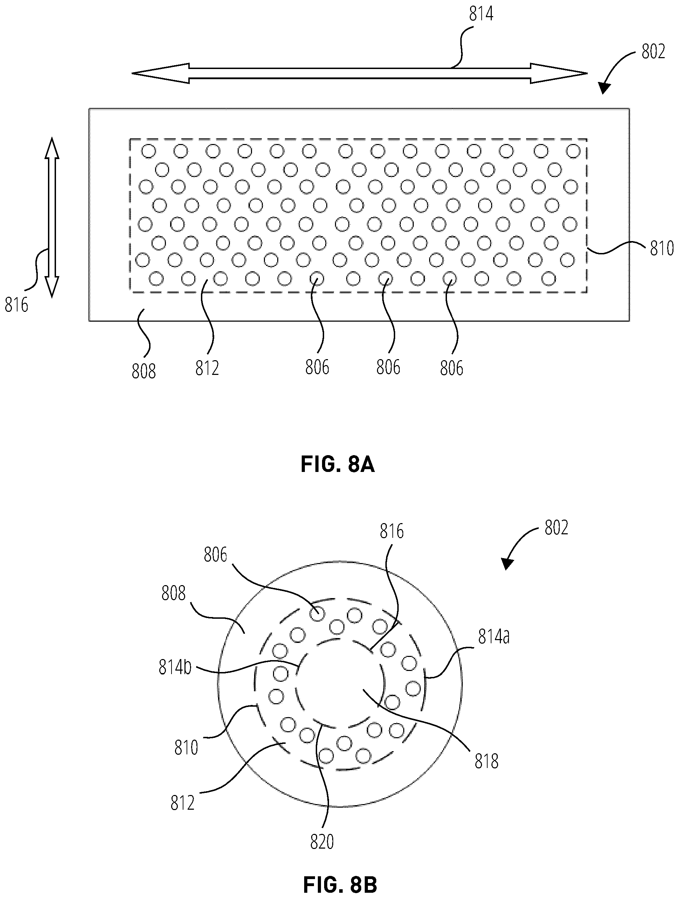

FIG. 8A and FIG. 8B show further example cell culture constructs 802 where the fibres 806 are arranged in an envelope 810 which has a high aspect ratio.

In particular, FIG. 8A and FIG. 8B show end views of the example cell culture constructs 802. In these examples, the fibres 806 are embedded in the end support 808 within an envelope 810. That is, the first ends of the fibres 806 are arranged within the envelope 810 in the plane of the end of the end support 808.

Within the envelope 810 the fibres 806 are arranged in a fibre distribution. The fibres 806 are spaced apart, such spaces are formed between the fibres 806 where they extend between the end support 808 and the second ends (not shown) of the fibres 806. The fibre distribution may be regular or irregular, and may have a minimum and/or maximum fibre spacing.

In the example of FIG. 8A the envelope 810 and the end support 808 are rectangular. The envelope 810 has a first dimension 814 and a second dimension 816. The first dimension 814 is greater than the second dimension 816. In the illustrated example, a ratio of the first dimension 814 to the second dimension 816 is about 3:1. In various examples the ratio may be at least 2:1, for example at least 2.5:1, for example at least 3:1, for example at least 3.5:1, for example at least 4:1, for example at least 5:1, for example at least 7:1, for example at least 8:1, for example at least 9:1, for example at least 10:1, for example at least 11:1, for example at least 12:1, for example at least 13:1, for example at least 14:1, for example at least 15:1, for example at least 16:1, for example at least 17:1, for example at least 18:1, for example at least 19:1, for example at least 20:1.

In larger scale examples the ratio may be even higher, for example at least 50:1, for example at least 100:1, for example at least 200:1, for example at least 300:1, for example at least 500:1, for example at least 500:1. In examples, the ratio may be at most 500:1, for example at most 750:1, for example at most 1000:1. In preferred examples the ratio may be between 2:1 and 1000:1, for example between 2:1 and 500:1, for example between 2:1 and 100:1, for example between 2:1 and 50:1. In examples, the ratio may be between 3:1 and 500:1, for example between 3:1 and 100:1, for example between 3:1 and 50:1.

In this way, the envelope 810 has high aspect ratio - one dimension is greater than the other dimension. This means that the fibres 806 are arranged in a bundle having a small thickness compared to its width relative to the direction of fluid flow in some examples.

Advantageously, the first dimension 814 is the dimension of the envelope 810 against which fluid flows during use (seeding, culturing and/or harvesting).

In the examples of FIG. 8B, the end support 808 is circular and the envelope 810 is formed as an annular region about a central channel 818. The central channel 818 extends from the end support 808 through the arrangement of the fibres 806.

The cell culture construct 802 may be made by potting the fibres 806 in the annular shape illustrated. Alternatively, the fibres 806 may be potted in a 2D array and then rolled into the annular shape illustrated, held in form by a cup or similar.

The central channel 818 may be used to pass fluid through the centre of the envelope 810 in the cell culturing system. In particular, a port may be connected to the central channel 818. Fluid may be passed into the central channel 818 such that it flows through a space surrounded by the fibres 806. In other examples, fluid may be sucked from the central channel 818, reversing the direction of fluid flow. The flow direction may be changed (reversed) during use.

A tube, for example a distribution tube 820, may extend through the central channel 818. The distribution tube 820 may be permeable or perforated, for example having a plurality of openings. The distribution tube 820 may act to support the fibres 806, holding them within the envelope 810. The distribution tube 820 may be removable from the central channel 818 or fixed therein.

The distribution tube 820 may include a plurality of openings along the length of the tube, each opening connecting the central channel 818 to the volume surrounding the fibres 806. In such examples a distribution of the openings may be arranged to control fluid flow into / out of the envelope 810 to / from the central channel 818. For example, there may be more and/or larger openings in the end of the distribution tube 820 closer to the second ends of the fibres 806 relative to the end support 808 such that fluid flowing into the central channel 818 from the end support 808 is more evenly distributed along the length of the cell culture construct 802.

The central channel 818 and/or distribution tube 820 may additionally or alternatively be used to house a sensor or probe, for example a dissolved oxygen (dO) sensor, a pH sensor, or a capacitance sensor.

The central channel 818 and/or distribution tube 820 may additionally or alternatively be used to house a heating device for heating the cell culture construct 802.

The central channel 818 and/or distribution tube 820 may additionally or alternatively be used for cleaning the cell culture construct 802. For example, a rotating spray ball, jet or spray head (for cleaning fluids and/or steam), or the like, may be passed into the central channel 818 or distribution tube 820 for cleaning the cell culture construct 802.

The central channel 818 and/or distribution tube 820 may additionally or alternatively be used in various harvesting techniques.

The envelope 810 has a first dimension 814a, 814b and a second dimension 816. The first dimension 814a, 814b is greater than the second dimension 816. The first dimension 814a may be the circumference of the outer boundary of the envelope 810 or the first dimension 814b may be the circumference of the inner boundary of the envelope 810, depending on the direction of fluid flow (from within the central channel 818, through the bundle of fibres 806 to the outside, or from the outside, through the bundle of fibres 806 into the central channel 818). In both cases, the second dimension 816 is the dimension through the bundle of fibres 806 against which fluid flows during use (seeding, culturing and/or harvesting). The first dimension 814a, 814b is the dimension through the bundle of fibres 806 in a direction perpendicular to the fluid flow direction.

The second dimension 816 is the thickness of the bundle of fibres 806 in the direction of fluid flow during use (in particular during seeding, culturing and harvesting).

In the illustrated example, a ratio of the first dimension 814a, 814 to the second dimension 816 is about 4:1. In various examples the ratio may be at least 2:1, for example at least 2.5:1, for example at least 3:1, for example at least 3.5:1, for example at least 4:1, for example at least 5:1, for example at least 10:1, for example at least 20:1. For larger scale cell culture construct 802 the ratio may be higher, for example at least 50:1, for example at least 100:1, for example at least 200:1, for example at least 300:1, for example at least 400:1, for example at least 500:1. In examples, the ratio may be at most 500:1, for example at most 750:1, for example at most 1000:1. In preferred examples the ratio may be between 2:1 and 1000:1, for example between 2:1 and 500:1, for example between 2:1 and 100:1, for example between 2:1 and 50:1. In examples, the ratio may be between 3:1 and 500:1, for example between 3:1 and 100:1, for example between 3:1 and 50:1.

In this way, the envelope 810 effectively has a high aspect ratio - one dimension is greater than the other dimension with respect to the fluid flow direction during use. This means that the bundle of fibres 806 has a small thickness compared to its width relative to the direction of fluid flow.

As explained in detail below, this ratio of the first dimension 814a, 814b to the second dimension 816 improves the seeding, culturing and harvesting using the cell culture construct 802.

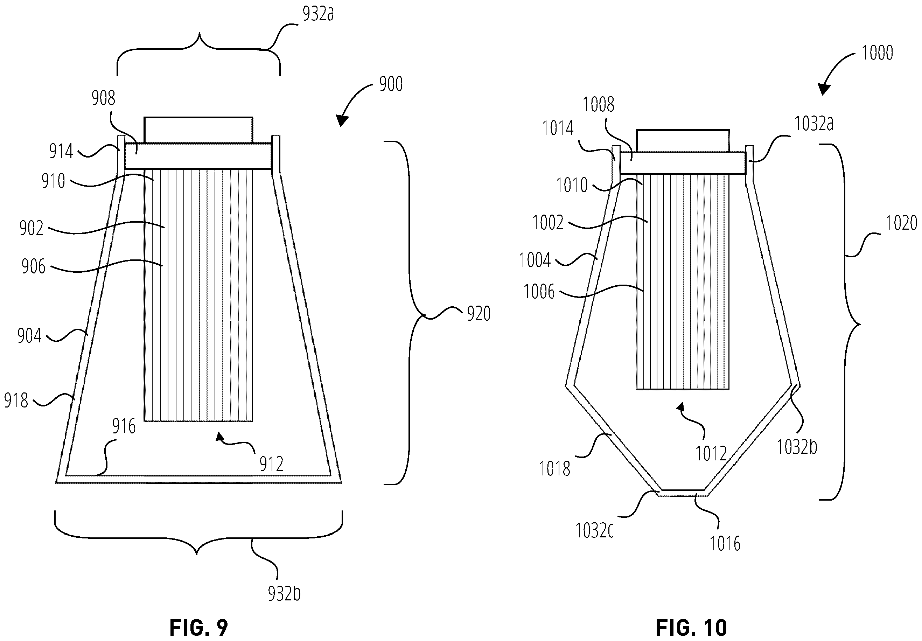

FIG. 9 and FIG. 10 illustrate example cell culturing systems 900, 1000 that are similar to those described with reference to FIG. 1 to FIG. 7B except that the culturing vessel 904, 1004 has a different shape.

In the examples of FIG. 1 to FIG. 6 the process vessel 104, 604 is illustrated as having a constant shape along its length. In particular, the process vessel 104 has a first vessel end 114, a second vessel end 116, and a vessel side wall 118 extending between the first vessel end 114 and the second vessel end 116. The process vessel 104 has a vessel length 120 between the first vessel end 114 and the second vessel end 116, and a vessel width 132. The vessel length 120 is the length of the process vessel 104 in a longitudinal direction between the first vessel end 114 and the second vessel end 116. In these examples, the process vessel 104 has a uniform cross-section along the length of the process vessel 104. In particular, the process vessel 104 has a constant vessel width 132. The process vessel 104 may have a square, rectangular, or circular cross-section, for example.

In contrast, in the examples of FIG. 9 and FIG. 10 the culturing vessel 904, 1004 has a non-constant vessel width between the first vessel end 914, 1014 and the second vessel end 916, 1016.

In particular, FIG. 9 illustrates a cell culturing system 900 that includes a cell culture construct 902 received within a culturing vessel 904. The cell culture construct 902 includes an end support 908 and a plurality of fibres 906 extending from the end support 908. First ends 910 of the fibres 906 are supported in the end support 908, and second ends 912 of the fibres 906 are free to move within the culturing vessel 904. The end support 908 is received and supported at the first vessel end 914 of the culturing vessel 904 such that the fibres 906 extend into the culturing vessel 904. Various fluid ports may be provided, as described previously, for cell seedings, culturing, and harvesting processes.

In this example the culturing vessel 904 has a first vessel end 914 and a second vessel end 916 opposite to the first vessel end 914. A vessel side wall 918 extends between the first vessel end 914 and the second vessel end 916. The culturing vessel 904 has a vessel length 920 between the first vessel end 914 and the second vessel end 916. The culturing vessel 904 has a first vessel width 932a at the first vessel end 914 and a second vessel width 932b at the second vessel end 916. The second vessel width 932b is greater than the first vessel width 932a. In particular, the width of the culturing vessel 904 increases between the first vessel end 914 and the second vessel end 916. The culturing vessel 904 may be a conical vessel, although in may have flat sides and therefore be a pyramid (e.g., a frustrum of a pyramid) with three, four, or more flat sides.

The larger second vessel width 932b at the second vessel end 916 advantageously provides more space for the second ends 912 of the fibres 906 to move into and through. This may be advantageous during seeding, culturing and harvesting. In addition, the fibres 906 have a length less than the vessel length 920 so that the second ends 912 of the fibres 906 do not contact the second vessel end 916.

It will be appreciated that the same larger second vessel width 932b may be provided for the process vessel 604 illustrated in FIG. 6, in which a plurality of cell culture constructs 602 are received in the same process vessel 604.

FIG. 10 also illustrates a cell culturing system 1000 that includes a cell culture construct 1002 received within a process vessel 1004. The cell culture construct 1002 includes an end support 1008 and a plurality of fibres 1006 extending from the end support 1008. First ends 1010 of the fibres 1006 are supported in the end support 1008, and second ends 1012 of the fibres 1006 are free to move within the process vessel 1004. The end support 1008 is received and supported at the first vessel end 1014 of the process vessel 1004 such that the fibres 1006 extend into the process vessel 1004. Various fluid ports may be provided, as described previously, for cell seedings, culturing, and harvesting processes.

In this example the process vessel 1004 has a first vessel end 1014 and a second vessel end 1016 opposite to the first vessel end 1014. A vessel side wall 1018 extends between the first vessel end 1014 and the second vessel end 1016. The process vessel 1004 has a vessel length 1020 between the first vessel end 1014 and the second vessel end 1016. The process vessel 1004 has a first vessel width 1032a at the first vessel end 914, a second vessel width 1032b at a position between the first vessel end 1014 and the second vessel end 1016, and a third vessel width 1032c at the second vessel end 1016. The second vessel width 1032b is greater than the first vessel width 1032a and the third vessel width 1032c. In particular, the width of the process vessel 1004 increases and decreases between the first vessel end 1014 and the second vessel end 1016. The longitudinal location of the second vessel width 1032b (the widest point of the process vessel 1004) may be aligned with, or close to, the second ends 1012 of the fibres 1006.

The larger second vessel width 1032b advantageously provides more space for the second ends 1012 of the fibres 1006 to move into and through. This may be advantageous during seeding, culturing and harvesting.

Furthermore, the narrowing of the vessel width to the second vessel width 1032b at the second vessel end 1016 may provide improved collection of cells for harvesting. In particular, during harvesting cells may be detached from the fibres 1006 and allowed to settle, for example by gravity sedimentation, at the second vessel end 1016. The narrowing of the process vessel 1004 to the second vessel end 1016 may act to increase the concentration of the cells at the second vessel end 1016, allowing them to be harvested more effectively, for example through a port provided in the second vessel end 1016 or the vessel side wall 1018 proximate to the second vessel end 1016.

In addition, the fibres 1006 have a length less than the vessel length 1020 so that the second ends 1012 of the fibres 1006 do not contact the second vessel end 1016.

It will be appreciated that the same vessel shape as illustrated in FIG. 10 may be provided for the process vessel 604 illustrated in FIG. 6, in which a plurality of cell culture constructs 602 are received in the same process vessel 604.

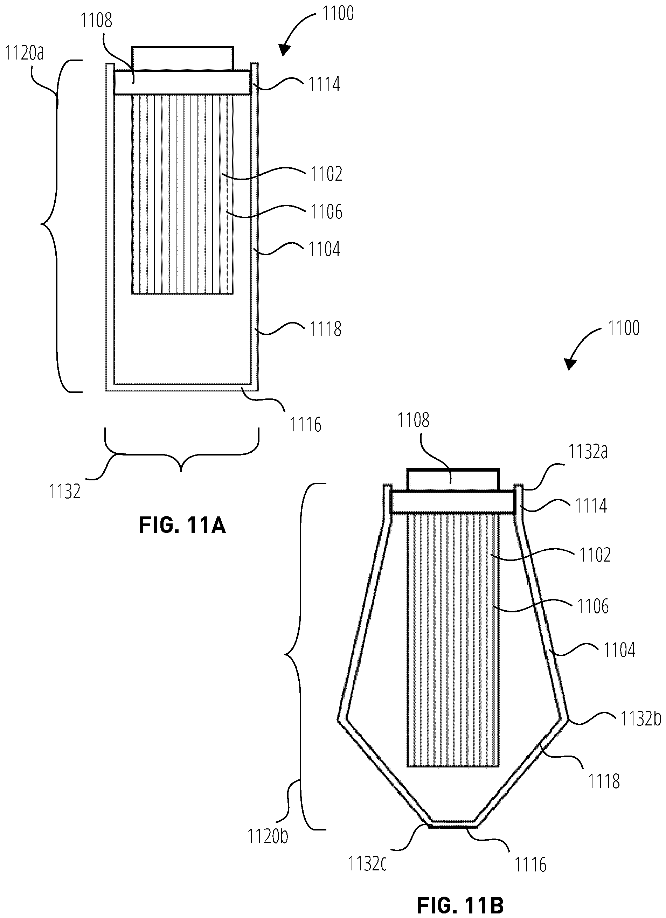

FIG. 11A and FIG. 11B show a further example cell culturing system 1100, which includes a cell culture construct 1102 received in a process vessel 1104 as per the examples described above. The cell culture construct 1102 includes a plurality of fibres 1106 extending from an end support 1108 so that second ends of the fibres 1106 are free to move within the process vessel 1104. The process vessel 1104 includes a first vessel end 1114 and a second vessel end 1116 and a vessel side wall 1118 extending between the first vessel end 1114 and the second vessel end 1116.

In this example, the process vessel 1104, in particular the vessel length 1120 (the distance between the first vessel end 1114 and the second vessel end 1116) is changeable. In particular, the process vessel 1104 can be changed from a first vessel length 1120a, shown in FIG. 11A, to a second vessel length 1120b, shown in FIG. 11B.

The vessel side wall 1118 is flexible or deformable to allow the first vessel end 1114 and the second vessel end 1116 to be moved relative to each other. In some examples, the vessel side wall 1118 may be flexible to permit changing between the configurations shown in FIG. 11A and FIG. 11B.

When changing the vessel length 1120, the vessel width 1132 may also be changed. For example, as shown in FIG. 11A the vessel width 1132 may have a first, constant value when the process vessel 1104 is in the first vessel length 1120a configuration, and when the process vessel 1104 is changed to the second vessel length 1120b configuration the vessel width 1132 may change. In the illustrated example, in the second vessel length 1120b configuration the process vessel 1104 has a first vessel width 1132a at the first vessel end 1114, a larger second vessel width 1132b at a position between the first vessel end 1114 and the second vessel end 1116, and a third vessel width 1132c at the second vessel end 1116. This is as described with reference to FIG. 10.

In one example, the vessel side wall 1118 is flexible. That is, the vessel side wall 1118 can be deformed. This allows the first vessel end 1114 to be moved relative to the second vessel end 1116 to change the vessel length 1120 and the vessel width 1132 as described. The second vessel end 1116 may be flexible or may be rigid, for example it may comprise a rigid support plate. The first vessel end 1114 may comprise a rigid support ring, or may be held in position by attachment to the end support 1108.

In an example, the process vessel 1104 may be placed in a support or mould that defines the shape of the vessel side wall 1118 and optionally also the second vessel end 1116. For example, the mould may be a cavity mould in which the process vessel 1104 can be placed. A first mould with an internal profile matching the arrangement of FIG. 11A may be provided so that when the process vessel 1104 is received in the first mould the vessel side wall 1118 is held in the illustrated form. A second mould with an internal profile matching the arrangement of FIG. 11B may be provided so that when the process vessel 1104 is received in the second mould the vessel side wall 1118 is held in the illustrated form. Accordingly, the shape of the process vessel 1104 may be changeable.

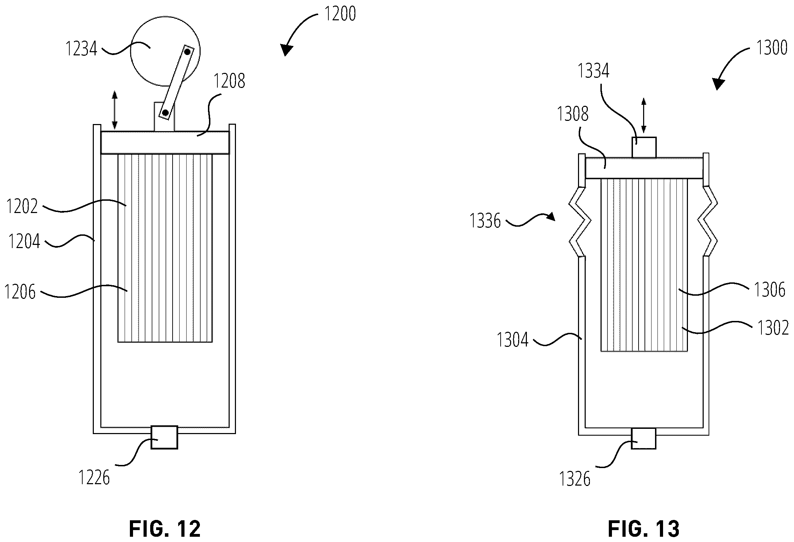

FIG. 12 illustrates an example moving mechanism 1234 that can generate movement of the process vessel 1204 relative to the fibres 1206. In this example, the cell culturing system 1200 includes a cell culture construct 1202 received in a process vessel 1204. The cell culture construct 1202 includes an end support 1208 and a plurality of fibres 1206 extending therefrom with second ends that are free to move within the process vessel 1204 as described previously. A port 1226 is provided in the process vessel 1204, which may be used for extracting cells and/or fluid, for example during harvesting.

The moving mechanism 1234 is attachable to the end support 1208 and operable to move the end support 1208 reciprocally relative to the process vessel 1204. The end support 1208 may be slidable within the process vessel 1204. The illustrated moving mechanism 1234 comprises a crank mechanism, but other mechanisms may be provided to generate reciprocal movement, for example a cam mechanism or a linear actuator.

The relative movement of the fibres 1206 and the process vessel 1204, and in particular the relative movement of the fibres 1206 in a fluid within the process vessel 1204, will cause the fibres 1206 to deform and for fluid shear to be applied to the fibres 1206. This may advantageously be used during seeding, to increase interactions between cells and the fibres 1206 to promote adhesion, or during harvesting, to dislodge adherent cells from the fibres 1206.

The example cell culturing system 1300 of FIG. 13 is similar to that of FIG. 12 except that the process vessel 1304, in particular the vessel side wall 1318, includes an extendible portion 1336 that allows the end support 1308 and fibres 1306 to be moved relative to a part of the process vessel 1304, generating relative movement of the fibres 1306 and a fluid within the process vessel 1304. A moving mechanism 1334 may be provided to move the end support 1308. The extendible portion 1336 may comprises a flexible or foldable section, such as a bellows section.

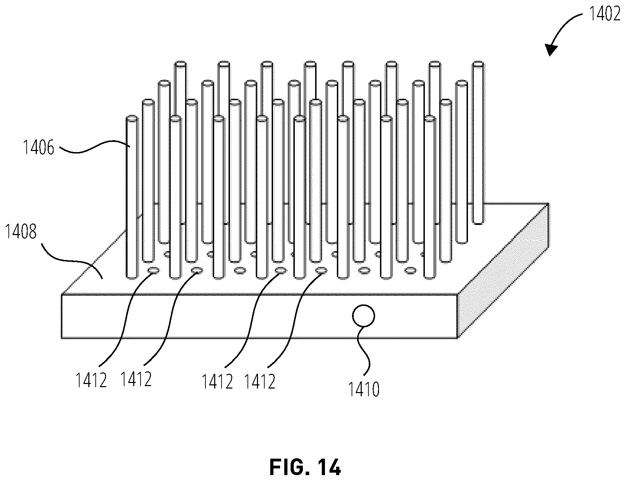

FIG. 14 illustrates an example cell culture construct 1402 for the cell culturing systems 100, 200, 300, 400, 500, 600, 700, 900, 1000, 1100, 1200, 1300 described above. In this example, cell culture construct 1402 includes an end support 1408 and a plurality of fibres 1406 extending from the end support 1408.

In this example, the end support 1408 includes a plurality of fluid outlets 1412. The fluid outlets 1412 are interspersed with the fibres 1406. The end support 1408 may include a manifold arrangement for connecting the fluid outlets 1412 to a side port 1410. The fluid supply systems described above may be connected with the side port 1410 for providing a fluid flow through the side port 1410 and fluid outlets 1412.

The fluid outlets 1412 may be used to generate a fluid flow along the fibres 1406, in particular along the fibres 1406 from the first vessel end towards the second vessel end, or vice versa. Such a fluid flow may directly apply fluid shear to cells adhered to the external surfaces of the fibres 1406 for detaching the cells during harvesting, or may directly provide an incident fluid flow of cell culture medium during culturing.

In other examples, gaseous bubbles may be created at fluid outlets 1412. For example, air, nitrogen, carbon dioxide, and/or oxygen may be pumped to the fluid outlets 1412 Such bubbles will promote cell detachment by shearing and/or scouring cells from the fibres 1406.

The different example cell culturing systems 100, 200, 300, 400, 500, 600, 700, 900, 1000, 1100, 1200, 1300 described above may be used for seeding, culturing and/or harvesting. In particular, the cell culturing systems 100, 200, 300, 400, 500, 600, 700, 900, 1000, 1100, 1200, 1300 described above may be used for all seeding, culturing and harvesting, within the same process vessel 104, 604, 704, 904, 1004, 1104, 1204, 1304. Alternatively or additionally, one or more of seeding, culturing and harvesting may be performed in a different vessel. For example, the cell culture construct 102, 602, 702, 902, 1002, 1102, 1202, 1302 may be moved between two or more vessels to perform different methods. In one example, the cell culture construct 102, 602, 702, 902, 1002, 1102, 1202, 1302 may be loaded into a seeding vessel for seeding, and/or the cell culture construct 102, 602, 702, 902, 1002, 1102, 1202, 1302 may be loaded into a culturing vessel for culturing, and/or the cell culture construct 102, 602, 702, 902, 1002, 1102, 1202, 1302 may be loaded into a harvesting vessel for harvesting. These vessels may be the same, or may be separate vessels. In one example the cell culture construct 102, 602, 702, 902, 1002, 1102, 1202, 1302 is loaded into a seeding and culturing vessel for seeding, and then removed and loaded into a harvesting vessel for harvesting. In another example, the cell culture construct 102, 602, 702, 902, 1002, 1102, 1202, 1302 may be loaded into a seeding vessel for seeding, then removed and loaded into a culturing and harvesting vessel for culturing and harvesting. In another example the cell culture construct 102, 602, 702, 902, 1002, 1102, 1202, 1302 may be loaded into a seeding vessel for seeding, then removed and loaded into a culturing vessel for culturing, then removed and loaded into a harvesting vessel for harvesting. Use of different vessels for different parts of the cell culturing method may permit greater efficiency, scale and repeatability of cell culturing.

In the seeding method, the cell culture construct 102, 602, 702, 902, 1002, 1102, 1202, 1302 is loaded into a seeding vessel. The seeding vessel may any one of the example process vessels 104, 604, 704, 904, 1004, 1104, 1204, 1304 described above. During seeding a seed cell suspension is provided to the seeding vessel, in particular to provide seed cells to the cell culturing system 100, 200, 300, 400, 500, 600, 700, 900, 1000, 1100, 1200, 1300.

The seed cell suspension may be provided by a fluid flow system, such as that described above. The fluid flow system may generate a fluid flow of the seed cell suspension through the seeding vessel. The fluid flow of seed cell suspension may be in a direction substantially parallel to the fibres, for example through the lumina of the fibres, in a direction from the first vessel end of the seeding vessel towards the second vessel end, and/or in a direction from the second vessel end of the seeding vessel towards the first vessel end, or circularly from the first vessel end, towards the second vessel end, and back towards the first vessel end. Flowing the seed cell suspension through the lumina of the fibres or along the fibres may provide a steady, controlled, relatively low flow rate of seed cells, with low fluid shear forces, to encourage seeding, for example adherence of cells to the fibres. In some examples, the flow of seed cell suspension may be from the second vessel end towards the first vessel end, within the extracapillary space in the seeding vessel. Due to the free second ends of the fibres, this fluid flow will cause the fibres to move and spread out, increasing interactions between the seed cells and the fibres, encouraging seeding of the cells. Applying suction to the lumina of the fibres during seeding may encourage cells to adhere to the external surfaces of the fibres. In other examples, the fluid flow of seed cell suspension may be transverse to the direction of the fibres, for example using side ports 134 as shown in FIG. 5. Such a flow direction will cause the fibres to move and increase interactions between the seed cells and the fibres, encouraging seeding of the cells. In examples, the fluid flow of the seed cell suspension may be reversible one or more times during the seeding method to help to ensure that seed cells are evenly spread throughout the seeding vessel, in particular over the fibres. In examples, the fluid flow of the seed cell suspension may be at a constant flow rate, or at a variable flow rate, for example at an intermittent flow rate. This may help to ensure that seed cells are evenly spread throughout the seeding vessel, in particular over the fibres.

In examples, the seeding vessel is rotated during seeding. The seeding vessel may be inclined during rotation. The rotation of the seeding vessel may be about a rotation axis that is parallel to the longitudinal direction of the seeding vessel. The seeding vessel may be loaded into a rotating assembly with an actuator for rotating the seeding vessel. Such rotation may encourage seed cells to evenly spread throughout the seeding vessel, in particular over the fibres. The rotation may happen at the same time as the fluid flow described above.

In some examples, the fibres may be moved relative to the seeding vessel during seeding, for example using a moving mechanism 1234, 1334 such as those described with reference to FIG. 12 and FIG. 13. The seed cell suspension may be provided in the seeding vessel, and the fibres moved within the fluid to encourage movement and separation of the fibres, and mixing of the seed cells within the fibres to help to encourage seed cells to evenly spread throughout the seeding vessel, in particular over the fibres.

In examples, the seed cells may seed on the fibres, in particular the seed cells may be adherent cells that adhere to the fibres, on internal and/or external surfaces of the fibres. In other examples, the seed cells may be adherent cells that adhere to other structures within the seeding vessel, for example fixed or floating scaffolds. In other examples, the seed cells may be non-adherent cells that remain in a suspension within the seeding vessel, in which case the seeding method provides for a substantially even distribution of cells within the fluid in the seeding vessel.

In the cell culturing method, the cell culture construct 102, 602, 702, 902, 1002, 1102, 1202, 1302 is loaded into a culturing vessel. The culturing vessel may any one of the example process vessels 104, 604, 704, 904, 1004, 1104, 1204, 1304 described above. During the cell culturing method a cell culture medium is provided to the culturing vessel to facilitate cell growth.

The cell culture medium may be provided by a fluid flow system, such as that described above. The fluid flow system may generate a fluid flow of the cell culture medium through the culturing vessel. The fluid flow of cell culture medium may be in a direction substantially parallel to the fibres, for example through the lumina of the fibres, in a direction from the first vessel end of the culturing vessel towards the second vessel end, and/or in a direction from the second vessel end of the culturing vessel towards the first vessel end, or circularly from the first vessel end, towards the second vessel end, and back towards the first vessel end. Flowing the cell culture medium through the lumina of the fibres or along the fibres may provide a steady, controlled, relatively low flow rate of cell culture medium, with low fluid shear forces, to encourage cell growth without dislodging or damaging cells or groups of cells. In some examples, the flow of cell culture medium may be from the second vessel end towards the first vessel end, within the extracapillary space in the seeding vessel. Due to the free second ends of the fibres, this fluid flow will cause the fibres to move and spread out, increasing interactions between the cell culture medium and the fibres. In other examples, the fluid flow of cell culture medium may be transverse to the direction of the fibres, for example using side ports 134 as shown in FIG. 5. Such a flow direction will cause the fibres to move and increase interactions between the cell culture medium and the cells, encouraging growth of the cells. In examples, the fluid flow of the cell culture medium may be reversible one or more times during the cell culturing method to help to ensure that nutrients are provided to cells throughout the culturing vessel. In examples, the fluid flow of the cell culture medium may be at a constant flow rate, or at a variable flow rate, for example at an intermittent flow rate. This may help to ensure that cell culture medium reaches cells throughout the culturing vessel.

In examples, the culturing vessel is rotated during the cell culturing method. The culturing vessel may be inclined during rotation. The rotation of the culturing vessel may be about a rotation axis that is parallel to the longitudinal direction of the culturing vessel. The culturing vessel may be loaded into a rotating assembly with an actuator for rotating the culturing vessel. Such rotation may encourage cell culture medium to evenly spread throughout the culturing vessel. The rotation may happen at the same time as the fluid flow described above.

In some examples, the fibres may be moved relative to the culturing vessel during the cell culturing method, for example using a moving mechanism 1234, 1334 such as those described with reference to FIG. 12 and FIG. 13. The cell culture medium may be provided in the culturing vessel, and the fibres moved within the fluid to encourage movement and separation of the fibres, and mixing of the cell culture medium within the fibres to help to encourage cell culture medium to evenly spread throughout the culturing vessel.

In examples, the cells may be adhered on the fibres, in particular on internal and/or external surfaces of the fibres. In these examples, cell culture medium may beneficially be circulated through the fibres themselves, in which case the cells will be supplied with nutrients directly, and through the porous walls of the fibres. In other examples, the cells may be adherent cells that adhere to other structures within the culturing vessel, for example fixed or floating scaffolds. In this case, effective circulation of the cell culture medium through the culturing vessel helps to ensure that all cells are supplied with nutrients for growth. In other examples, the cells may be non-adherent cells that remain in a suspension within the culturing vessel, in which case the cell culturing method provides for a substantially even flow of cell culture medium within the culturing vessel.

In the harvesting method, the cell culture construct 102, 602, 702, 902, 1002, 1102, 1202, 1302 is loaded into a harvesting vessel. The harvesting vessel may any one of the example process vessels 104, 604, 704, 904, 1004, 1104, 1204, 1304 described above. During the harvesting method a harvesting fluid flow may be provided to the harvesting vessel to facilitate detachment and/or collection of the cells.

The harvesting fluid may be provided by a fluid flow system, such as that described above. The fluid flow system may generate a fluid flow of the harvesting fluid through the harvesting vessel. The fluid flow of harvesting fluid may be in a direction substantially parallel to the fibres, for example through the lumina of the fibres, in a direction from the first vessel end of the seeding vessel towards the second vessel end, and/or in a direction from the second vessel end of the harvesting vessel towards the first vessel end, or circularly from the first vessel end, towards the second vessel end, and back towards the first vessel end. Flowing the harvesting fluid through the lumina of the fibres or along the fibres may provide a controlled fluid shear effect for detaching cells from the fibres without damaging the cells or groups of cells. In some examples, the flow of harvesting fluid may be from the second vessel end towards the first vessel end, within the extracapillary space in the harvesting vessel. Due to the free second ends of the fibres, this fluid flow will cause the fibres to move and spread out, increasing interactions between the harvesting fluid and the fibres. In other examples, the fluid flow of harvesting fluid may be transverse to the direction of the fibres, for example using side ports 134 as shown in FIG. 5. Such a flow direction will cause the fibres to move and increase interactions between the harvesting fluid and the cells, encouraging detachment and entrainment of the cells. In examples, the fluid flow of the harvesting fluid may be reversible one or more times during the harvesting method to help to ensure that cells are detached and/or entrained from all parts of the harvesting vessel. In examples, the fluid flow of the harvesting fluid may be at a constant flow rate, or at a variable flow rate, for example at an intermittent flow rate. This may help to ensure that harvesting fluid reaches cells throughout the harvesting vessel.

In examples, the harvesting vessel is rotated during the harvesting method. The harvesting vessel may be inclined during rotation. The rotation of the harvesting vessel may be about a rotation axis that is parallel to the longitudinal direction of the harvesting vessel. The harvesting vessel may be loaded into a rotating assembly with an actuator for rotating the harvesting vessel. Such rotation may encourage harvesting fluid to evenly spread throughout the harvesting vessel. The rotation may happen at the same time as the fluid flow described above.

In some examples, the fibres may be moved relative to the harvesting vessel during the harvesting method, for example using a moving mechanism 1234, 1334 such as those described with reference to FIG. 12 and FIG. 13. The harvesting fluid may be provided in the harvesting vessel, and the fibres moved within the fluid to encourage movement and separation of the fibres, and mixing of the harvesting fluid within the fibres to help to encourage cell detachment and entrainment.

In examples, the cells may be adhered on the fibres, in particular on internal and/or external surfaces of the fibres. In these examples, harvesting fluid may beneficially be circulated through the fibres themselves, in which case the harvesting fluid will cause detachment of the cells by directly flowing over the cells and also passing through the porous walls of the fibres. In other examples, the cells may be adherent cells that adhere to other structures within the harvesting vessel, for example fixed or floating scaffolds. In this case, effective circulation of the harvesting fluid through the harvesting vessel helps to ensure that all cells are detached and/or entrained. In other examples, the cells may be non-adherent cells that remain in a suspension within the harvesting vessel, in which case the harvesting fluid provides for a substantially even flow to entrain the cells for extraction.

The harvesting method may comprise harvesting the cells themselves, for example for use a food product or as a cell gene therapy product. Harvesting of the cells themselves may include detachment of the cells from whatever structure they may be adhered to, and entrainment of the cells for collection. The harvesting method may include concentrating the cells in a suspension and extraction from the harvesting vessel. Concentrating the cells may comprise directing the cells into a narrower part of the harvesting vessel, for example by gravity sedimentation towards a narrow second vessel end of the harvesting vessel as shown in FIG. 10 and FIG. 11B. The cells can be extracted through a port, such as the second port in the second vessel end as described above.

In other examples, the harvesting method comprises harvesting a cell product produced by the cells during the cell culturing method. In these examples, the cell product may be within the fluid in the culturing vessel or harvesting vessel, and the harvesting method may include extracting, filtering, concentrating and/or mixing to harvest the cell product.

In examples, the harvesting fluid may comprise water, phosphate buffered saline (PBS), enzyme solution in a suitable buffer, and/or chemical agents in solution.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

## Claims

1. A cell culture construct for a cell culturing system, the cell culturing system having a process vessel with an interior volume and being adapted to receive the cell culture construct,
wherein the cell culture construct comprises a plurality of fibres and an end support, wherein first ends of the plurality of fibres are supported in the end support such that the plurality of fibres extend from the end support and into the interior volume of the process vessel when the cell culture construct is received in the process vessel, and
wherein second ends of the plurality of fibres are disposed within the interior volume of the process vessel.

2. The cell culture construct of claim 1, wherein the second ends of the plurality of fibres are free to move within the process vessel.

3. The cell culture construct of claim 1 or 2, wherein the plurality of fibres are hollow, openended fibres, or wherein the plurality of fibres are solid fibres.

4. The cell culture construct of any one of claims 1 to 3, wherein the plurality of fibres are inedible.

5. A cell culturing system comprising:
a process vessel, and
the cell culture construct of any one of claim 1 to claim 4, wherein the cell culture construct is received in the process vessel such that the plurality of fibres extend into the interior volume of the process vessel and the second ends of the plurality of fibres are disposed within the interior volume of the process vessel.

6. The cell culturing system of claim 5, wherein the process vessel comprises a first vessel end, a second vessel end, and a vessel side wall between the first vessel end and the second vessel end, and wherein the end support is disposed at the first vessel end.

7. The cell culturing system of claim 6, wherein the process vessel has a vessel length between the first vessel end and the second vessel end, and wherein the plurality of fibres have a length less than the vessel length.

8. The cell culturing system of claim 6 or 7, comprising a fluid flow system operable to provide a fluid flow through the process vessel.

9. The cell culturing system of claim 8, wherein the plurality of fibres comprise hollow, openended fibres, and wherein the fluid flow system is adapted to generate a fluid flow through the plurality of fibres.

10. The cell culturing system of claim 9, wherein the fluid flow system comprises a first port fluidly connected to lumina of the plurality of fibres to input fluid through the fibres.

11. The cell culturing system of claim 9 or 10, wherein the fluid flow system comprises a second port in communication with an internal volume of the process vessel.

12. The cell culturing system of any one of claims 8 to 11, wherein the fluid flow system is configured to generate a fluid flow in a direction between the first vessel end and the second vessel end, or vice versa.

13. The cell culturing system of any one of claims 8 to 12, wherein the fluid flow system comprises side ports disposed in the vessel side wall.

14. The cell culturing system of any one of claims 6 to 13, wherein the process vessel has a vessel length between the first vessel end and the second vessel end, and a vessel width in a transverse direction, and wherein the vessel width increases and/or decreases between the first vessel end and the second vessel end.

15. The cell culturing system of any one of claims 5 to 14, comprising a moving mechanism for moving the process vessel and/or the cell culture construct to generate movement of the plurality of fibres within the process vessel.

16. A cell culturing method for culturing cells in the cell culturing system of any one of claims 5 to 15, wherein the cell culturing system comprises a fluid flow system, and wherein the method comprises:
providing cells in the process vessel, and
passing a cell culture medium through the process vessel to culture the cells.

17. The cell culturing method of claim 16, comprising circulating fluid from the first ends of the plurality of fibres, to the second ends of the plurality of fibres, and extracting fluid from the process vessel.

18. A harvesting method for harvesting cells and/or cell products from the cell culturing system of any one of claim 5 to claim 15, the harvesting method comprising generating a fluid flow through the process vessel and collecting cells and/or cell products.

19. A seeding method for seeding cells in the cell culturing system of any one of claim 5 to claim 15, the seeding method comprising generating a fluid flow of a seed cell suspension through the process vessel.

20. A method of culturing cells, the method comprising:
the seeding method of claim 19,
the cell culturing method of claim 16 or claim 17, and
the harvesting method of any of claim 18.

21. The method of claim 20, wherein the seeding method and/or the cell culturing method, and/or the harvesting method are carried out in the same process vessel or in different process vessels.

22. The method of any of any one of claims 16 to 21, wherein the method is a method of culturing a food product, and wherein the cells comprise food product cells, for example animal cells and/or animal cell precursors, or plant cells, or fungal cells, or bacterial cells, optionally wherein animal cells or animal cell precursors comprise muscle cells, fat cells, adipocyte cells, or cell precursors, including fibroblasts, skeletal muscle cells, smooth muscle cells, and/or myoblasts.

23. The method of any of any one of claims 16 to 21, wherein the method is a method of culturing cells for production of a biochemical, biopharmaceutical, cosmetic, biomass, cell, or fermentation product.

24. The method of any of claim 23, wherein the harvesting method is a method of harvesting the cultured cells and/or a method of harvesting a cell product.
